# EUROPEAN PATENT SPECIFICATION

(11) **EP 3 865 170 B1**
(45) Date of publication and mention of the grant of the patent: **15.02.2023**
(21) Application number: 21167033.6
(22) Date of filing: 28.12.2017
(51) Int. Cl.: A61M 16/14, A61M 11/04

(54) **AEROSOL DELIVERY SYSTEM**
AEROSOLABGABESYSTEM
SYSTÈME D'ADMINISTRATION D'AÉROSOL

(30) Priority: 29.12.2016 US 201662440273 P
(43) Date of publication of application: 18.08.2021
(62) Divisional of application: 17828765.2
(73) Proprietor: Philip Morris Products S.A., 2000 Neuchâtel (CH)
(72) Inventor: ATTERBURY, William, Columbus, OH 43235 (US); HASSENPFLUG, Eric, Westerville, OH 43081 (US); HAUBERT, Thomas D., Columbus, OH 43214 (US); HOOKS, Larry Keith, Jr., Columbus, OH 43230 (US); KO, Michael, Brookline, MA 02445 (US); LEAMON, James, Warminster, PA 18974 (US); LORENZ, Michael, Gahanna, OH 43230 (US); MORA, Ludwin, Columbus, OH 43221 (US); O'LOUGHLIN, Jane, Galloway, OH 43119 (US); PRESCOTT, James A., Columbus, OH 43235 (US); SCHMITT, Stephen C., Dublin, OH 43017 (US); SOMOGYE, Ryan, Grove City, OH 43123 (US); ULRICH, Michael Scott, Columbus, OH 43204 (US); WEINSTEIN, Lawrence A., Warminster, PA 18974 (US); WILDER, Steve, Blacklick, OH 43004 (US)
(74) Representative: Millburn, Julie Elizabeth

(56) References cited:
- WO-A1-2015/177044
- WO-A1-2015/177253
- GB-A- 2 504 732
- US-A1- 2015 245 669

## Description

This disclosure relates to an aerosol delivery system for delivery of aerosol from an aerosol delivery unit, which is fed a liquid formulation from a syringe pump unit. The aerosol delivery system can also include a power control unit and operation interface. A carrier gas, such as a pressurized inspiratory gas, can be used to carry aerosol generated from the aerosol delivery unit to a patient.

Patients, both adult and infants, in respiratory failure or those with respiratory dysfunction are often mechanically ventilated in order to provide suitable rescue and prophylactic therapy. Mechanical ventilation can be invasive and non-invasive. Non-invasive ventilation can be provided to a patient capable of spontaneous breathing. A ventilatory circuit for administering non-invasive continuous positive airway pressure ventilation ("CPAP") includes a positive pressure generator connected by tubing to a patient interface, such as a mask, nasal prongs, or an endotracheal tube, and an exhalation path, such as tubing that allows discharge of the expired gases, for example, to the ventilator.

Producing medical aerosols with the use of a heated capillary generator have been disclosed in U.S. Patent No. 8,251,055 and WO2014/029827 A1. The ventilation gas tube, expiratory flow tube and entrained aerosol tube can be connected to the patient interface via an aerosol delivery connector, for example, as disclosed in WO 2009/117422 A2.

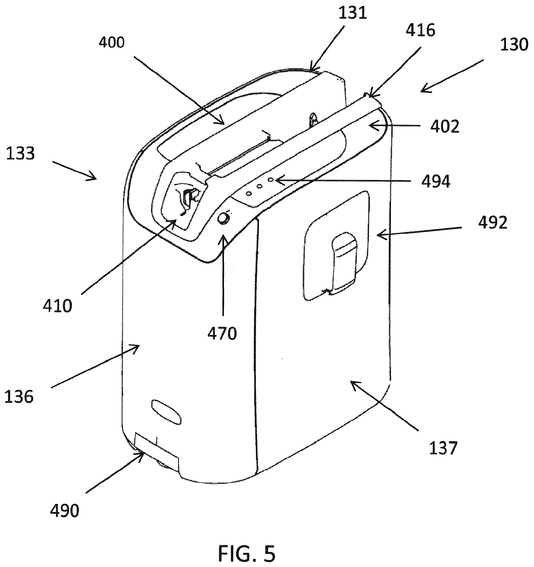

GB 2 504 732 A discloses an assembly for evaporating a volatile fluid 202, the assembly comprising a device 1 and a refill 2 which are detachable from one another. The device 1 comprises a perforating element 105 and a magnetic induction coil 103 configured to operate with an alternating current passed therethrough at a frequency of between 20KHz and 500KHz. The refill 2 comprises a reservoir 201 for the volatile fluid 202 with a sealable pierceable cover 206 and a magnetic susceptor 204 arranged to heat the material 202 predominately by magnetic hysteresis when said alternating current is passed through the induction coil. Optionally the refill may further comprise a wick 203 to draw the fluid from the reservoir 201. A design for the refill 2 is shown in Figure 5B which uses a wick 203. In this case, the wick is shaped to sit in the bottom of the reservoir 201, and is pre-saturated with volatile material 202. The susceptor 204 is preferably placed within the wick 203. When the susceptor 204 is heated by the induction coil 103, the volatile material 202 near the susceptor starts to evaporate from the wick 203. As this volatile material evaporates, volatile material 202 located further away from the susceptor 204 diffuses towards it through capillary action in the wick 203. In another design for the refill 2 shown in Figure 5C at least a portion of the wick 203 extends above the volatile material 202 in the reservoir 201. As material evaporates from the wick 203, new volatile material enters the wick 203 from the reservoir 201. The new volatile material diffuses towards the susceptor 204 through capillary action in the wick 203.

Further aerosol delivery systems are known for example from US2015/245669 A1, WO2015/177044 A1 and WO2015/177253 A1.

A cartridge assembly is disclosed, the cartridge assembly comprising: an active part of the cartridge assembly including a capillary tube; a susceptor, the susceptor configured to partially surround the capillary tube; and a pair of displaceable covers, which surround at least the capillary tube and the susceptor.

According to the invention there is provided an aerosol delivery system, the aerosol delivery system comprising: an aerosol delivery unit, the aerosol delivery unit comprising: a cartridge receiver, which is configured to receive a cartridge assembly; and an inductor configured to receive the cartridge assembly and heat a liquid formulation within a capillary tube to produce an aerosol by induction heating; and a pump, the pump comprising: a container holder configured to receive a container containing the liquid formulation, and wherein the container holder is configured to pivot about an axis to expose an opening on a bottom of the container holder; a plunger for applying a force to push the liquid formulation out of a distal end of the container into a proximal end of the capillary tube; and a pressure jacket configured to support the container within the container holder.

A method is disclosed of producing an aerosol, the method comprising: placing a cartridge assembly into a cartridge receiver of an aerosol delivery unit, the cartridge assembly including (a) an active part of the cartridge comprising (i) a capillary tube, (ii) a susceptor configured to at least partially surround the capillary tube, (iii) a handle, and (vi) an insulator, the handle and the insulator arranged on an upper surface of the susceptor, and (b) a pair of displaceable covers, which at least partially surround the insulator the capillary tube and the susceptor; and displacing the pair of displaceable covers by moving the active part of the cartridge into an inductor.

The disclosure is explained below with reference to the exemplary embodiments shown in the drawings. In the drawings:
FIG. 1 is a perspective view of an aerosol delivery system in accordance with an exemplary embodiment.
FIG. 2 is a perspective view of the aerosol delivery system as shown in FIG. 1 in accordance with an exemplary embodiment.
FIGS. 3A-3C are a side view, a front view, and another side view, respectively, of the aerosol delivery system as shown in FIG. 1 in accordance with an exemplary embodiment.
FIG. 4 is a perspective view of a power control unit of the aerosol delivery system in accordance with an exemplary embodiment.
FIG. 5 is a perspective view of an aerosol delivery unit of the aerosol delivery system in accordance with an exemplary embodiment.
FIG. 6 is a perspective view of a portion of the aerosol delivery unit as shown in FIG. 5.
FIG. 7A is an exploded perspective view of a portion of the aerosol delivery unit in accordance with an exemplary embodiment.
FIG. 7B is a perspective view of a pair of locking pins in accordance with an exemplary embodiment.
FIG. 8A is a perspective view of a potted inductor of the aerosol delivery unit in accordance with an exemplary embodiment.
FIG. 8B is a perspective view of cartridge receiver of the aerosol delivery unit in accordance with an exemplary embodiment.
FIG. 8C is a perspective view of another portion of the aerosol delivery unit in accordance with an exemplary embodiment.
FIG. 9A is a perspective view of a disposable cartridge assembly in the user interface of the aerosol delivery unit in accordance with an exemplary embodiment.
FIG. 9B is a perspective view of a transition adaptor cover placed over the transition adaptor of the aerosol delivery unit in accordance with an exemplary embodiment.
FIG. 10 is a perspective view of a portion of the disposable cartridge assembly and the aerosol delivery unit in accordance with an exemplary embodiment.
FIG. 11 is a perspective view of a portion of the disposable cartridge assembly in accordance with an exemplary embodiment.
FIG. 12 is an exploded perspective view of a portion of the disposable cartridge assembly in accordance with an exemplary embodiment.
FIG. 13 is a perspective view of a portion of the disposable cartridge assembly in accordance with an exemplary embodiment.
FIG. 14 is another perspective view of a portion of the disposable cartridge assembly in accordance with an exemplary embodiment.
FIG. 15A is a perspective view of a cover of the disposable cartridge assembly in accordance with an exemplary embodiment.
FIG. 15B is a perspective view of the right and left covers of the disposable cartridge assembly in accordance with an exemplary embodiment.
FIG. 15C is another perspective view of view of the right and left covers of the disposable cartridge assembly in accordance with an exemplary embodiment.
FIG. 15D is a further perspective view of view of the right and left covers of the disposable cartridge assembly in accordance with an exemplary embodiment.
FIG. 16A is a perspective view of an inner portion of the disposable cartridge assembly in accordance with an exemplary embodiment.
FIG. 16B is a perspective view of a portion of the disposable cartridge assembly in accordance with an exemplary embodiment.
FIG. 17 is a cross-sectional view of a portion of the disposable cartridge assembly and transition adapter in accordance with an exemplary embodiment.
FIG. 18 is a perspective view of the transition adapter in accordance with an exemplary embodiment.
FIG. 19 is a cross-sectional view of the transition adapter in accordance with an exemplary embodiment.
FIG. 20 is a cross-sectional view of the transition adapter in accordance with an exemplary embodiment.
FIG. 21 is a cross-sectional view of the transition adapter in accordance with an exemplary embodiment.
FIG. 22 is a cross-sectional view of a tube set assembly in accordance with an exemplary embodiment.
FIG. 23 is a perspective view of the syringe pump unit in accordance with an exemplary embodiment.
FIG. 24 is a perspective view of the syringe pump unit in accordance with an exemplary embodiment.
FIG. 25 is a perspective view of the syringe pump unit in accordance with an exemplary embodiment.
FIG. 26 is a perspective view of the syringe pump unit in accordance with an exemplary embodiment.

Aerosols are useful in drug delivery. For example, it is often desirable to treat respiratory ailments with, or deliver drugs by means of, aerosol sprays of fine, dispersed particles of liquid, or solid, or liquid and solid, for example, pharmaceuticals, and so forth, which are inhaled and delivered into a patient's lungs. Aerosols can be generated by a heated capillary aerosol generator (CAG) by feeding a liquid formulation into a heated capillary tube or passage (referred to herein as "capillary", "heated capillary" or "heated capillary tube") while heating the capillary sufficiently such that the liquid formulation is at least partially volatilized, so that upon discharge from the heated capillary, the liquid formulation is in the form of an aerosol. The length of the capillary (or capillary tube) can depend on heat requirements dictated by, among other factors, the composition of the aerosol to be generated.

As used herein, the term "aerosol" refers to liquid or solid particles that are suspended in an air or gas. The "aerosol" or "aerosolized agent" referred to herein contains one or more of the active agents, as referred to below.

The term "ventilation" or "respiratory ventilation" as used herein refers to external mechanical or artificial support of a patient's breathing. The overall goals of mechanical ventilation are to optimize gas exchange, patient work of breathing and patient comfort while minimizing ventilator-induced lung injury. Mechanical ventilation can be delivered via positive-pressure breaths or negative-pressure breaths. Additionally, the positive-pressure breaths can be delivered noninvasively or invasively. Noninvasive mechanical ventilation (NIMV) generally refers to the use of a mask or nasal prongs to provide ventilatory support through a patient's nose, or a patient's mouth, or a patient's nose and mouth. The most commonly used interfaces for noninvasive positive pressure ventilation are nasal prongs, nasopharyngeal tubes, masks, or nasal masks. NIMV can be distinguished from those invasive mechanical ventilatory techniques that bypass the patient's upper airway with an artificial airway (endotracheal tube, laryngeal mask airway or tracheostomy tube). NIMV can be provided by either bi-level pressure support (so called "BI-PAP") or continuous positive airway pressure (CPAP).

The employment of mechanical ventilation, whether invasive or non-invasive, involves the use of various respiratory gases, as would be appreciated by the skilled artisan. Respiratory gases are sometimes referred to herein as "CPAP gas", "ventilation gas", "ventilation air", "inspiratory flow", "expiratory flow", or simply "air." As used herein, the terms "ventilation gas", "air", "oxygen", "medicinal gas" and "gas" are used interchangeably to refer to a ventilation gas or flow driven oxygen/air and include any type of gas normally used for respiratory therapy. The term "ventilator" referred to herein can also be described as a source of ventilation gas. A carrier gas is used to carry aerosolized drugs in administering respiratory therapy. The term "carrier gas" can be used herein interchangeably with the term "entrainment gas" or "sheath gas" and includes any type of gas normally used for respiratory therapy as disclosed above.

A ventilation circuit for administering positive pressure ventilation includes a positive pressure generator or a source of positive end expiratory pressure (PEEP valve or column of water) connected by tubing to a patient interface, such as a mask, nasal prongs, or an endotracheal tube, and an exhalation path, such as tubing that allows discharge of the expired gases, for example, to the ventilator such as a constant flow CPAP or to an underwater receptacle as for "bubble" CPAP. The inspiratory and expiratory tubes can be connected to the patient interface via a "Y" connector or an aerosol delivery connector, for example, as disclosed in WO 2009/117422 A2, which contains a port for attaching each of the inspiratory and expiratory tubes, as well as a port for the aerosol, the patient interface and, a port for attaching a pressure sensor.

The aerosol generated by the heated capillary or other means is known to be mixed with a carrier gas or sheath gas for transporting to the patient. Admixing the aerosol and sheath gas in a transition adapter is disclosed, for example, in U.S. Patent Publication Nos. 2008/0110458 A1 and 2014/0053831 A1.

In an exemplary embodiment, the source of the carrier gas is a ventilator, which is used for providing ventilator support to the patient receiving aerosolized drug. In another embodiment, the source of the carrier gas is independent from a ventilator and is coming from a source of a positive flow. Preferably, the carrier gas is non-humidified. In accordance with an exemplary embodiment, the non-invasive breathing support can be provided to preterm infants with irregular breathing or lung diseases with continuous positive airway pressure (CPAP) given via nasal prongs or a mask. In accordance with an exemplary embodiment, it would be desirable to treat infants with an aerosol generated by an aerosol delivery system and carried by a carrier gas, such as a non-humidified gas to a patient.

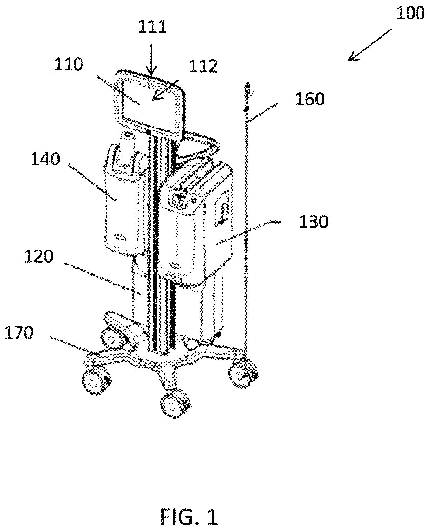

FIG. 1 is a perspective view of an aerosol delivery system 100 in accordance with an exemplary embodiment. As shown in FIG. 1, the aerosol delivery system 100 can include an operator's interface unit (or touch screen interface) 110, a power control unit (or power controller) 120, an aerosol delivery unit 130, and a syringe pump unit (or syringe pump) 140. In addition, each unit 110, 120, 130, 140 can include a microprocessor or microcomputer having an operating system to carry out the processes as disclosed herein. The units 110, 120, 130, 140 are configured to be individually replaced, or serviced, or replaced and serviced, if necessary.

As shown in FIG. 1, for mobility, each of the units 110, 120, 130, 140, can be configured to be attachable to a wheeled pole or therapeutic cart 170. In addition, the system 100 can include a temperature probe 160. In accordance with an exemplary embodiment, a prominent light bar 111, for example, can be arranged at the top of the system 100, for example, on the touchscreen interface 110, and can communicate system status and can be visible, for example, if the operator is across the room and not looking directly at the display screen 110.

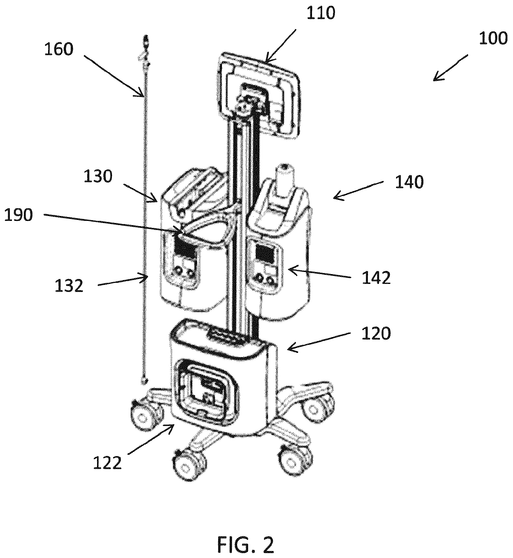

FIG. 2 is a perspective view of the aerosol delivery system 100 as shown in FIG. 1 in accordance with an exemplary embodiment. As shown in FIG. 2, the operator's interface unit 110, the power control unit (or power controller) 120, the aerosol delivery unit 130, and the syringe pump unit 140, can have a separate or dedicated user interface, 112, 122, 132, 142, for preparing and operating each of the individual units 110, 120, 130, 140, or an entirety of the system 100, or each of the individual units 110, 120, 130, 140 and an entirety of the system 100. In accordance with an exemplary embodiment, each unit 110, 120, 130, 140 can have an operator's interface, such as, for example, an on/off button, touch screen, switch, latch, loading controls, which communicates with one or more of the other units 110, 120, 130, 140.

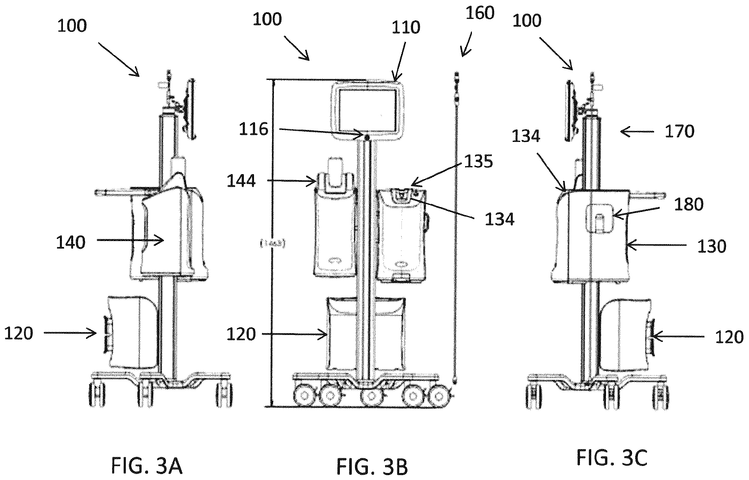

FIGS. 3A-3C is a side view, a front view, and another side view of the aerosol delivery system 100 as shown in FIG. 1 in accordance with an exemplary embodiment. In accordance with an exemplary embodiment, the working heights and positioning of the touchscreen interface or display unit 110, the syringe holder 144 of the syringe pump unit 140, the top cover of the cartridge receiver 134 and corresponding aerosol tubing connection area 135 of the aerosol delivery unit 130 can be optimized for the intended user's height and to facilitate administering treatment through the use of the therapeutic cart or wheeled pole 170.

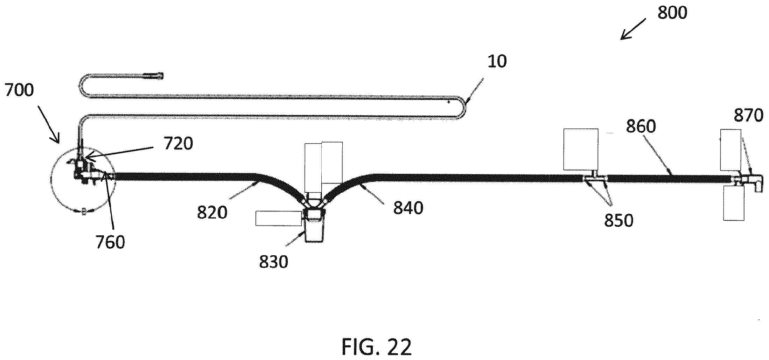

In accordance with an exemplary embodiment, a prominent color highlights the primary user interface area on each unit 110, 120, 130, 140. For example, the body color of each unit 110, 120, 130, 140 can be predominantly white to show cleanliness and blend in with the surrounding environment. In accordance with an exemplary embodiment, on a side of the aerosol delivery unit 130, a hook feature (or cavity with a prong or hook) 180 can be incorporated for hanging/storing the aerosol tubing 800 (FIG. 22) and the temperature probe 160 when the system 100 is in storage and waiting for use. In accordance with an exemplary embodiment, wire/cable connectors (not shown) for each unit 110, 120, 130, 140 can be color-coded to facilitate proper connection during assembly. In accordance with an exemplary embodiment, the therapeutic cart or wheeled pole 170 can have a handle 190 to help assist an operator or user to move or change the location of the cart or wheeled pole 170 within a patient's room, or the hospital, or a patient's room and the hospital.

In accordance with an exemplary embodiment, as shown in FIGS. 1-3C, the operator's interface unit (or display screen) 110 can be a touchscreen display system, which displays a treatment status to the operator and provides controls for operating the system through, for example, an interactive Graphical User Interface (GUI). The operator's interface unit 110 can also include an ON/OFF button or control 116 (FIG. 3B). In accordance with an exemplary embodiment, a light bar 111 can be arranged at the top of the system 100, for example, on the touchscreen interface 110, and can communicate system status and can be visible, for example, if the operator is across the room and not looking directly at the display screen 110.

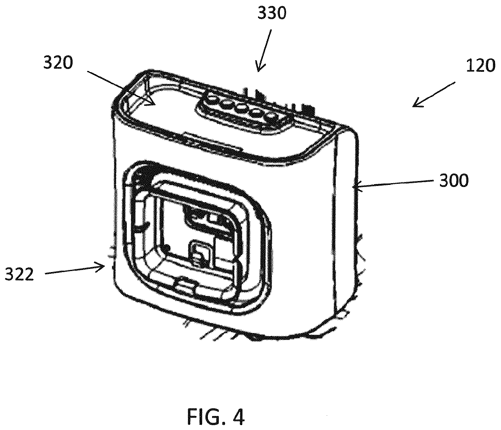

FIG. 4 is a perspective view of a power control unit (or power controller) 120 of the aerosol delivery system 100 in accordance with an exemplary embodiment. The power control unit has a power input and a power output 322. As shown in FIG. 4, the power control unit 120 including a housing 300, which is arranged to house or provide, for example, one or more sources of power, for example, a 12 volt supply, or a 48 volt supply, or a 12 volt supply and a 48 volt supply, a plurality of wire harnesses configured to supply power, for example, 12 volts of direct current, or 48 volts of direct current, or 12 volts of direct current and 48 volts of direct current, a power cord, a printed circuit board (PCB) panel, a display screen, an embedded computer or processor, and optionally, a fan. In accordance with an exemplary embodiment, the power control unit 120 can be plugged into an electrical source of power, or alternatively, the power control unit 120 can be battery powered or have an alternative source of power which powers the power supply in case an electrical source of power is not available.

In accordance with an exemplary embodiment, as shown in FIG. 4, the power control unit (or power controller) 120 can include a recess 320 on a top (or an upper surface) for storing extra disposable components and an integral cord wrap for storing and securing the power cord during storage. As shown in FIG. 4, on the top or upper surface of the housing 300 of the power control unit 120, a plurality of wire harnesses 330 are provided to connect the power control unit 120, for example, to the operator's interface unit 110 (FIG. 1), the aerosol delivery unit 130, and the syringe pump unit 140. The power control unit 120 can include an on/off switch, and a USB port for data exchange and software updates.

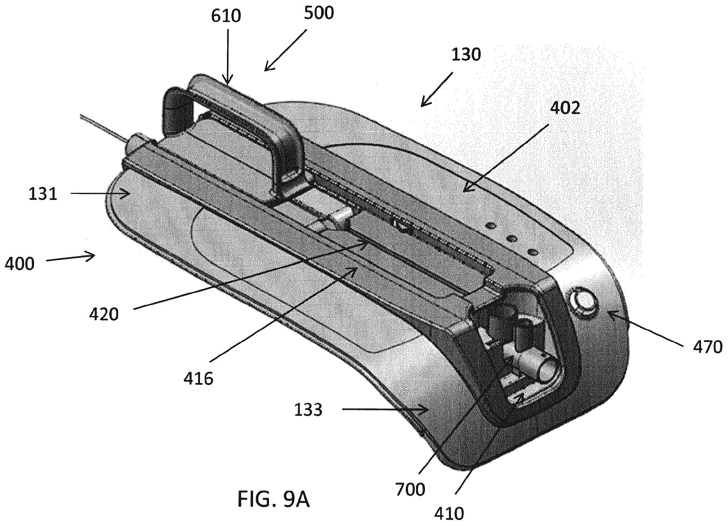

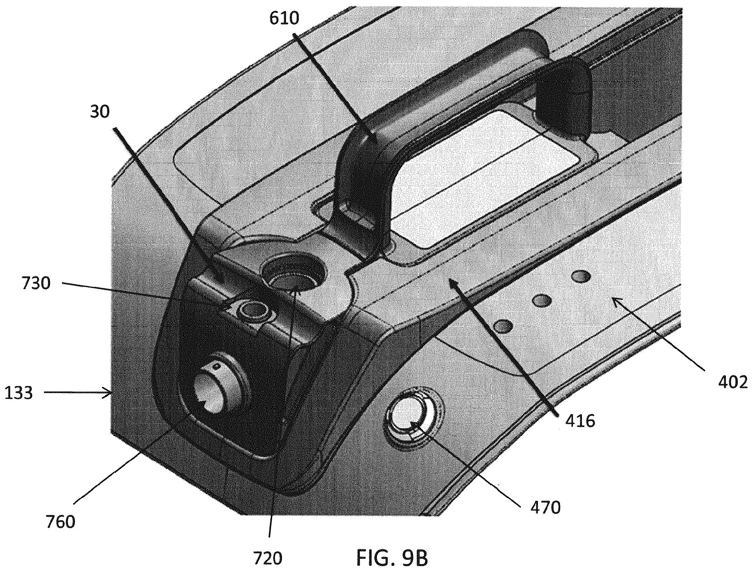

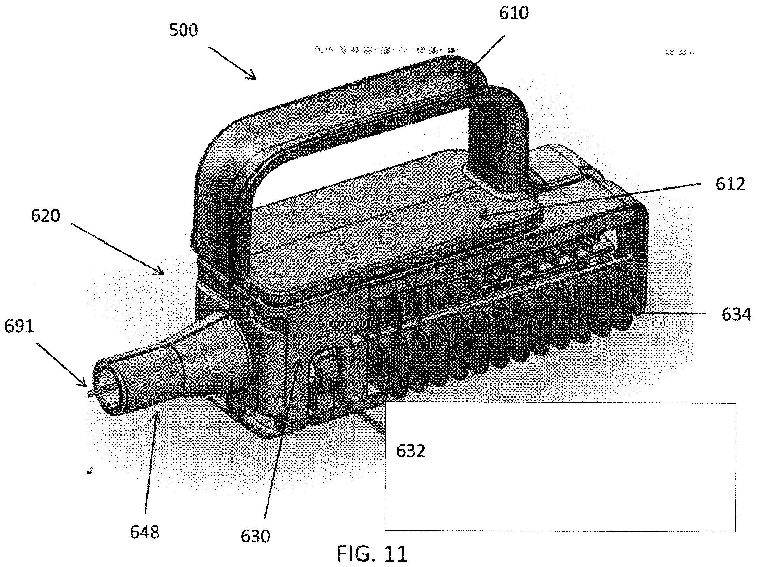

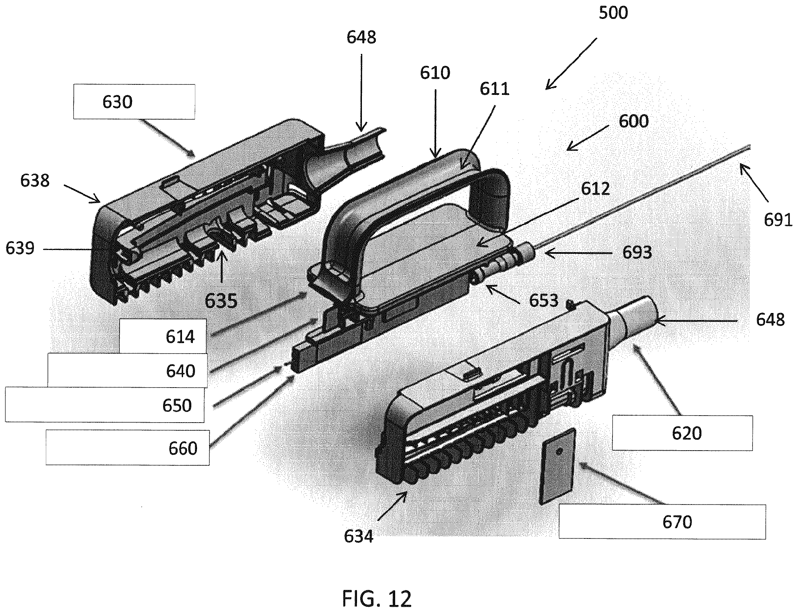

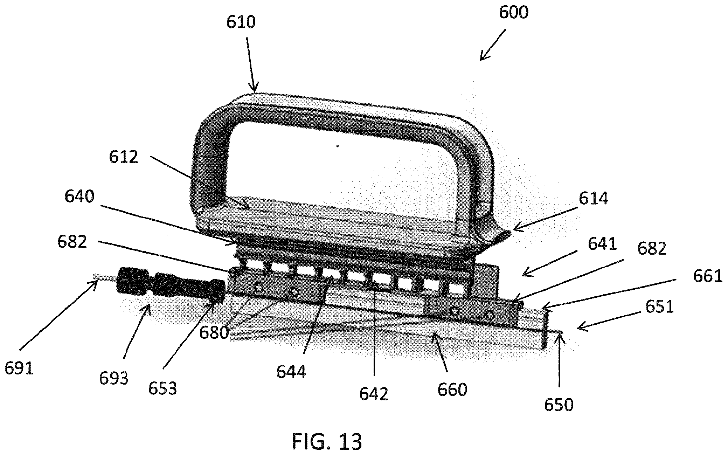

FIG. 5 is a perspective view of an aerosol delivery unit 130 of the aerosol delivery system 100 (FIG. 1) in accordance with an exemplary embodiment. As shown in FIG. 5, the aerosol delivery unit 130 includes a cartridge receiver 400 which is configured to receive (1) a cartridge assembly (or cartridge) 500 (FIGS. 11 and 12), (2) a disposable aerosol tube set 800 (FIG. 22) which includes a transition adapter 700 (FIGS. 9A and 9B), and (3) a temperature probe 160 (FIGS. 1-3C). In accordance with an exemplary embodiment, the cartridge assembly 500 is preferably a one-time use or disposable cartridge. The aerosol delivery unit 130 can have a connection 470 (FIGS. 9A and 9B), for example, a temperature sensor jack for the temperature probe 160 on the top surface or cover 402. In addition, the aerosol delivery unit 130 can include a cup holder (or placement) 490 for a condensation trap (not shown) on a front panel 136, and tube storage 492 on a side panel 137. In an alternative embodiment, the temperature can be shown as a light bar or digital display, which can provide a signal to a user if the system 100 is working within a desired temperature range during use. Lights 494 on the top surface or cover 402 of the aerosol delivery unit 130 can provide feedback to the user regarding the unit's status. In accordance with an exemplary embodiment, a user makes the physical connection between a distal end of the capillary 651 (FIG. 13) and a transition adapter 700 (FIGS. 9A and 9B) on the cartridge receiver 400 by placing the active part of the cartridge 600 into a designated slot in the cartridge receiver 400.

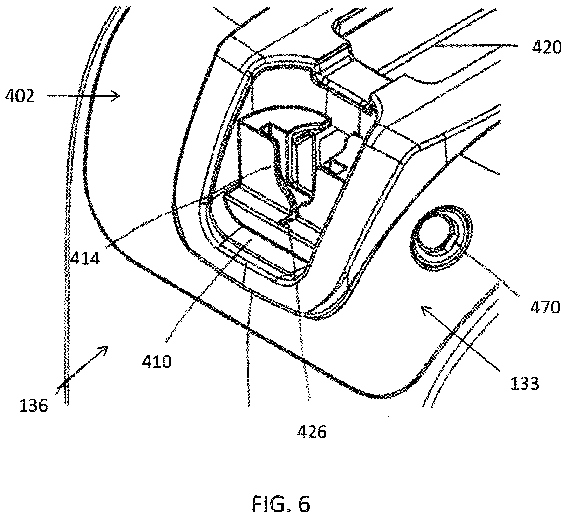

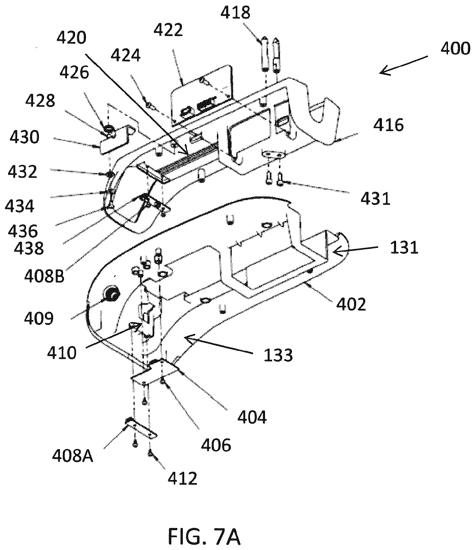

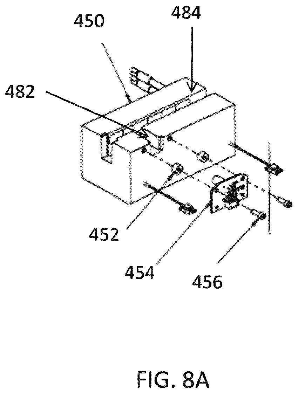

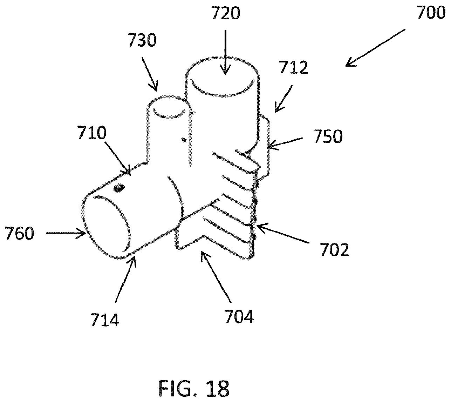

FIG. 6 is a perspective view of the aerosol delivery unit 130 (FIG. 1) showing a portion of FIG. 5 on a distal end 133 of the cartridge receiver 400. As shown in FIG. 6, the distal end 133 of the aerosol delivery unit 130 includes an aerosol output pocket 410, an active alignment groove 420 configured to guide an active part of the disposable cartridge (or unshielded part of the cartridge) 600 into a potted inductor 450 (FIG. 8A), a transition adapter cavity 414, and a transition adapter placement groove 426, which connects with the transition adapter flag 704 (FIG. 18) such that when the flag 704 is inserted into the transition adapter placement groove 426, the light can be blocked, and a sensor 408A (FIG. 7A) signals that the transition adapter 700 has been placed in the cartridge receiver 400 (as shown, for example, in FIG. 9A).

FIG. 7A is an exploded perspective view of a portion of the aerosol delivery unit 130, a cartridge receiver 400 in accordance with an exemplary embodiment. As shown in FIG. 7A, the cartridge receiver 400 can include a top surface or cover 402 and a platform receiver 416. Alternatively, the cartridge receiver 400 can be made of a single part or more than two parts. The cartridge receiver 400 can include an optical sensor assembly (for example, an LED printed circuit board (PCB) assembly) 404 for displaying an operational status of the aerosol delivery unit 130, a first sensor PCB assembly 408A configured to sense the presence of the transition adapter 700 (FIGS. 9A and 9B) on the distal end 133 (FIGS. 9A and 9B) of the aerosol delivery unit 130 during use, and a second sensor PCB assembly 408B configured to detect the engagement of the capillary's tip with the transition adapter 700. A plurality of screws 406 can be used to attach the optical sensor assembly (or LED PCB assembly) 404 to the top cover 402. A plurality of screws 412 can be used to attach the first sensor PCB assembly 408A to the top cover 402. In addition, the top cover 402 can include a wire harness (including a connector) 409 for a temperature sensor 160 (FIG. 1). Hardware 438 can be used to attach the second sensor assembly 408B to the platform receiver 416.

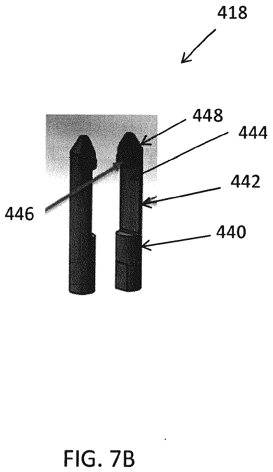

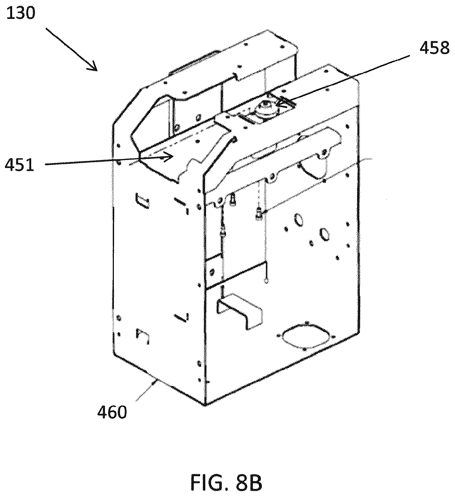

In accordance with an exemplary embodiment, the cartridge receiver 400 can also include, near the proximal end 131 (FIG. 5) of the aerosol delivery unit 130, an item serving as a placement for the cartridge as the cartridge is first inserted into the cartridge receiver 400. As shown in FIGS. 7A and 7B, a pair of placement pins 418 is configured to receive the cartridge 500 (FIG. 9A). The placement pins 418 can be attached to the platform receiver 416 by hardware 431. A cartridge identification (ID) PCB assembly 422 works together with a data card 670 (FIG. 12) to identify a cartridge 500 as new (or previously used) as well as to provide any other useful information and can be attached to the platform receiver 416 by hardware 424. A cartridge latch assembly 430 is configured to be actuated by a cartridge lock 458 (FIG. 8B) in the cartridge receiver 400. The cartridge latch assembly 430 can be attached to the platform receiver 416 by hardware 426, 428, 432, 434, and 436.

FIG. 7B is a perspective view of a pair of placement pins 418 in accordance with an exemplary embodiment. As shown in FIG. 7B and further described in detail below with reference to FIG. 9A and 9B, each of the placement pins 418 is configured to disengage the covers (to allow the active part of the cartridge 600 (FIG. 13) to move forward) once the disposable cartridge 500 is nested into the cartridge receiver 400 and keeps the covers 620, 630 in place, or locked, or in place and locked, into the cartridge receiver 400 while the active part of the cartridge 600 is moved forward into the heating area (or inductor 450) and until the active part of the cartridge 600 is fully returned into the pair of covers 620, 630 (FIG. 11). As shown in FIG. 7B, each of the placement pins 418 has a generally cylindrical shape 440 with an inner cavity 442 having a relatively flat or planar surface 444 and a flange 446 on an upper end 448 of each pin 418. The inner cavity 442 of the placement pins is configured to allow the active part of the cartridge 600 freely move in and out of the covers.

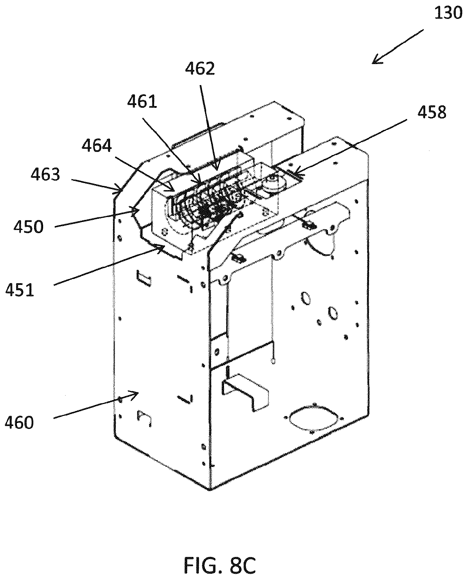

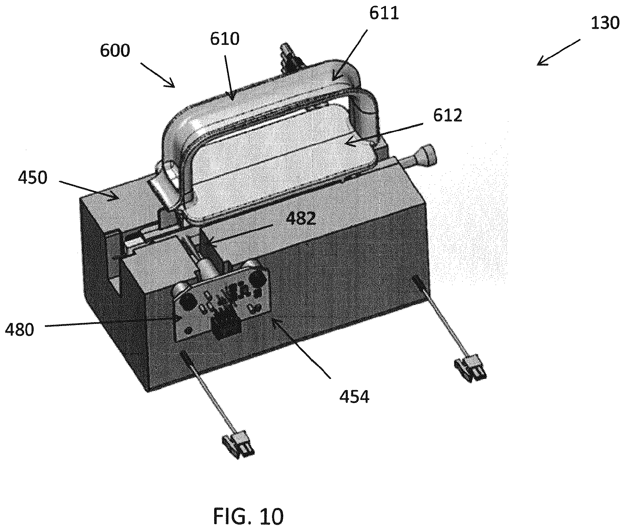

FIGS. 8A, 8B, and 8C are perspective views of another portion of the aerosol delivery unit 130 in accordance with an exemplary embodiment. As shown in FIG. 8A, the aerosol delivery unit 130 can include a heating unit such as a potted inductor 450. The inductor 450 includes an induction coil 462 (FIG. 8C) for heating the susceptor 660 (FIG. 13). The inductor 450 can be placed in the potted inductor holder 451 of the housing 460 (FIG. 8B) attached by screws or otherwise. As shown in FIG. 8A, the inductor 450 can include an assembly having an infrared (IR) sensor board 454 with an IR sensor 480 (see FIG. 10) placed in a groove 482 allowing the IR sensor 480 to sense the temperature of the susceptor 660. The IR sensor board 454 can be held in place and adjacent to the inductor 450 by hardware such as, for example, a pair of spacers 452 and a pair of bolts 456. The inductor 450 also includes a groove 484 configured to fit with the active alignment groove 420 (FIG. 7A) in the platform receiver and accommodate the susceptor 660.

As shown in FIG. 8B, the aerosol delivery unit 130 includes a housing 460 configured to receive the inductor 450. After the inductor 450 is fitted in the frame (or housing) 460 within the potted inductor holder 451 at the distal end 133 of the aerosol delivery unit, the groove 484 (FIG. 8A) is aligned with the active alignment groove 420 and the placement pins 418 in the platform receiver 416 (see FIG. 7A). The housing 460 also includes the cartridge lock 458, which locks the disposable cartridge 500 within cartridge receiver 400.

FIG. 8C is a perspective view of another portion of the aerosol delivery unit 130 in accordance with an exemplary embodiment. As shown in FIG. 8C, the potted inductor 450 is configured to be received in a distal portion (or front end) 463 of the housing 460. The inductor 450 includes an induction coil 462, which heats the susceptor 660 (FIG. 13). In accordance with an exemplary embodiment, the susceptor 660 is positioned inside of the inductor 450, which is composed of an induction coil 462, which can include a wire 461 wound around at least one graphite core 464, and more preferably wound around a plurality of graphite cores 464. The current in the wire 461 is modulated typical of an induction heating process by an inductor control unit (not shown), which is housed within the aerosol delivery unit 130. Most inductive heaters heat the susceptor 660 directly. However, in accordance with an exemplary embodiment, the graphite cores 464 of the inductor 450 couple the susceptor 660 to the inductor coil 462 instead.

FIG. 9A is a perspective view of a top portion of the aerosol delivery unit 130 with a cartridge 500 inserted into in the cartridge receiver 400 in accordance with an exemplary embodiment. As shown in FIG. 9A, the cartridge 500 can initially be placed onto the placement pins 418 (not shown) within the cartridge receiver 400 at a proximal end (or backside) 131 of the aerosol delivery unit 130 with a handle 610 remaining visible. This position of the cartridge 500 within the cartridge receiver 400 will be referred to as a starting position. The transition adapter 700 is placed at the distal end 133 (or front side) within the aerosol output pocket 410 and adjacent to the active alignment groove 420.

FIG. 9B shows the transition adaptor cover 30 placed over the transition adaptor 700. On the top cover 402, a temperature sensor jack 470 can be arranged, such that an operator or user can determine if the aerosol delivery unit 130 is operating within the working temperature range of, for example, from approximately 150°C to 300°C. Alternatively, rather than a temperature sensor jack 470, the jack can be replaced with a light or visual display, which can signal if the system 100 is working within the working temperature range.

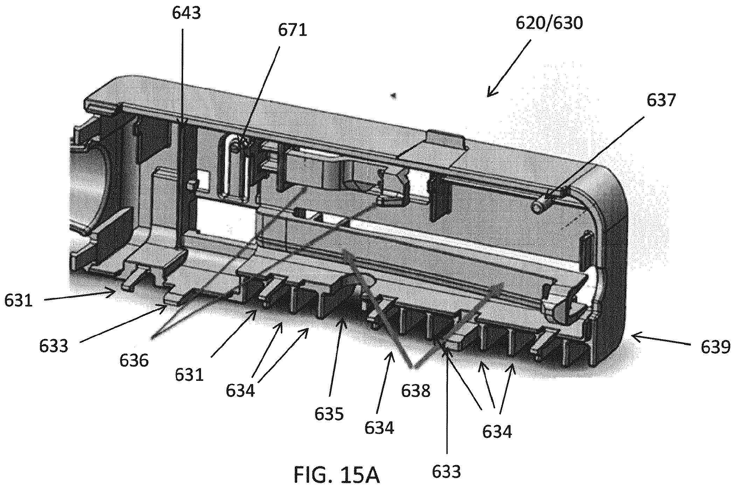

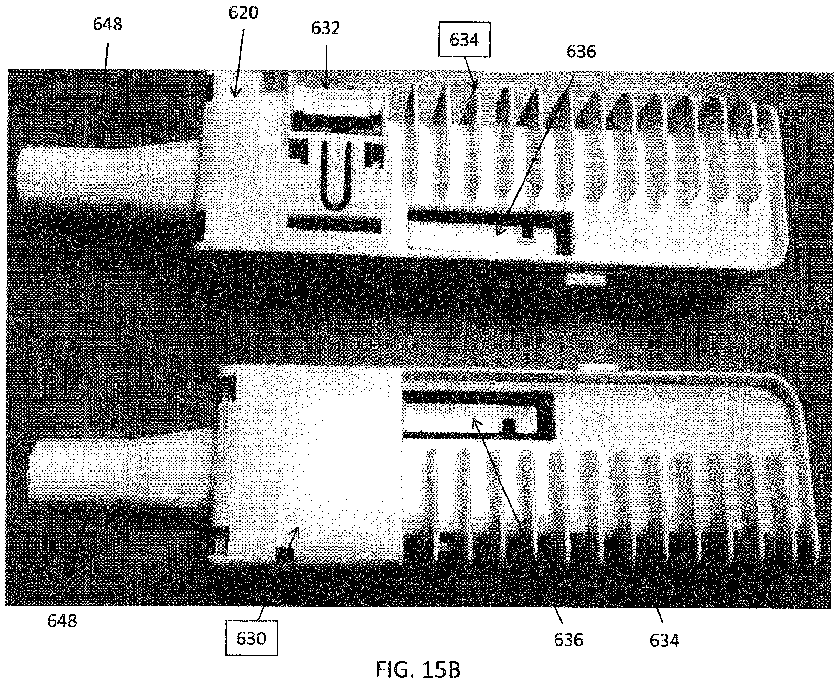

In accordance with an exemplary embodiment, for example, upon insertion of the cartridge 500 into the starting position in the cartridge receiver 400 as shown in FIG. 9A, the placement pins 418 (or a comparable other means) located within the cartridge receiver 400 move the latches 636 (see FIGS. 15A and 15B) in the covers 620, 630 of the cartridge 500, releasing the active part of the cartridge 600 (FIG. 13) from the cartridge 500. In accordance with an exemplary embodiment, the active part of the cartridge 600 (or unshielded portion) includes the insulator 640, the capillary tube 650, and the susceptor 660 (See FIGS. 12 and 13). The operator can then move the active part of the cartridge 600 by the handle 610 forward within the cartridge receiver 400 into an engaged or active position on a distal end or front portion 133 of the aerosol delivery unit 130 as shown in FIG. 9B for heating the susceptor 660 and thereby, heating the capillary tube 650 and aerosolizing the liquid formulation.

In accordance with an exemplary embodiment, the aerosol delivery system 100 is configured to interact with a device identifying card (or device ID card) 670 (see FIG. 12) on the cartridge 500 to determine, among other things, whether the cartridge 500 is new or previously been used. If the cartridge 500 has been used, the system 100 will not initiate or begin treatment. When a cartridge 500 has been used, the system 100 can mark the card 670 on the cartridge 500 as "read" electronically to prevent its re-use. The system 100 can also use the device ID card 670 to detect the presence of the cartridge 500 within the cartridge receiver 400.

In accordance with an exemplary embodiment, sensor 408A (see FIG. 7A) in the aerosol delivery unit 130 detects when the active part of the cartridge 600 is fully forward in the engaged position. In addition, as set forth above, the treatment or delivery of a drug will not begin until the active part of the cartridge 600 is in the fully engaged position. Once treatment has been initiated, the cartridge lock 458 (see FIGS. 8A and 8B) closes to fix the position of the active part of the cartridge 600 within the cartridge receiver 400. In accordance with an exemplary embodiment, the active part of the cartridge 600 cannot be removed while the lock 458 is activated and the lock 458 will not open until treatment has been completed, stopped, or paused. The cartridge latch assembly 430 is moved into a locking position by the lock 458 electronically. The lock 458 includes a motor with a cam attached to the motor shaft within the aerosol delivery unit 130.

In accordance with an exemplary embodiment, as disclosed above, the system 100 (FIG. 1) uses an induction heating system, in which temperature of the capillary 650 and the susceptor 660 can be monitored via an infrared (IR) sensor 480 (see FIGS. 8A and 10) on the infrared (IR) sensor board or assembly 454. In addition, both heating and sensing can be accomplished without direct physical contact with the active part of the cartridge 600.

In accordance with an exemplary embodiment, the induction heating system (FIG. 10) can provide evenly distributed heat to the drug delivery path and facilitate aerosolization. For example, in accordance with an exemplary embodiment, the drug temperature increases, for example, from room temperature to over 200°C in less than 2 seconds. The drug delivery path passes through a capillary tube 650, which is embedded inside a conductive element, referred to as the susceptor 660 (FIGS. 12 and 13). In accordance with an exemplary embodiment, the inductor 450 via induction heats the susceptor 660. Conductive, usually metallic, materials can be used to manufacture the susceptor 660.

In accordance with an exemplary embodiment, the temperature of the susceptor 660 can be monitored via an IR sensor 455 in the IR sensor board 454 (FIG. 10) and this signal can be used in a feedback control loop to precisely control the temperature of the susceptor 660. In accordance with an exemplary embodiment, a black coating can be applied to the susceptor 660 to provide a more consistent measurement surface, which could otherwise introduce variation.

FIG. 10 is a perspective view of a portion of the cartridge, the active part of the cartridge (or the unshielded part of the cartridge) 600 inserted into the inductor 450 for heating in accordance with an exemplary embodiment. As shown in FIG. 10, the active part of cartridge 600 is configured to be received within the potted inductor 450, which includes an IR sensor board or assembly 454 having an IR sensor 480 for sensing the temperature range of the susceptor 660 during use, for example, 150°C to 300°C, preferably, 200°C to 250°C. In accordance with an exemplary embodiment, the active part of cartridge 600 can be self-aligning to the transition adapter 700, as the transition adapter 700 has some limited freedom to move about during engagement.

In FIG. 10, the active part of the cartridge 600 (the cartridge without the covers 620, 630) with the handle 610 and its flat plate portion 612 are shown along with the rest of the active part of the cartridge 600 being inside of the inductor. The groove 482 provides visibility to the IR sensor PCB assembly 454. Another groove 484 provides the alignment with the susceptor 660 (FIG. 13) in part, for the inductor 450 to properly heat the susceptor 660.

FIG. 11 is a perspective view of a portion of the cartridge 500 in accordance with an exemplary embodiment. The cartridge assembly 500 can include a handle 610 and a relatively flat base plate 612, which is adjacent to a pair of covers 620, 630, for example, a right cover 620 and a left cover 630, an insulator 640, a capillary tube 650, a sleeve for tubing 648, tubing 691 for delivery of liquid drug into the capillary tube 650, and a susceptor 660. As shown in FIG. 11, the right and left covers 620, 630 can include a molded spring 632, which pushes or forces the cartridge and device identifying card (on opposite side) 670 (FIG. 12) against the cartridge identification PCB assembly 422 located on the aerosol delivery unit 130. Each of the right and left covers 620, 630 can include a plurality of heat dissipating fins 634, which also provide a space between the users grip of the handle 610, and the sometimes hot contents within the covers 620, 630 after use, for example, the capillary tube 650 and the susceptor 660.

FIG. 12 is an exploded perspective view of a disassembled cartridge 500 in accordance with an exemplary embodiment. As shown in FIG. 12, the cartridge 500 can include an oval or rectangular shaped handle 610 with a groove 611, the right cover 620, the left cover 630, an insulator 640, the capillary tube 650, the susceptor 660, the device identifying card 670, a sleeve for tubing 648, and optionally, the tubing 691 connected to the proximal end of the capillary for the delivery of the liquid drug to the capillary tube.

In accordance with an exemplary embodiment, it would be desirable that the mechanical features of the cartridge 500 are compact and protect the user from contacting with the heated capillary tube 650 and the susceptor 660 as shown in FIG. 12. For example, the covers 620 and 630 of the cartridge 500 can include heat dissipating features (for example, fins) 634 (FIGS. 15A-15D), which dissipate heat and creates space between the user and the hot components, for example, the heated capillary tube 650 and susceptor 660.

In accordance with an exemplary embodiment, the capillary tube 650 inside the cartridge 500 is part of the drug delivery path of the aerosol delivery system 100. In accordance with an exemplary embodiment, a new cartridge 500 is used for each drug treatment or for a portion of a drug regimen. The old (or used) cartridge 500 is removed and discarded, and a new cartridge 500 is installed into the aerosol delivery system 100. During treatment, for example, the capillary tube 650 and the susceptor 660 in the cartridge 500 can get very hot, for example, nearly 250°C. Therefore, upon removing and while the cartridge 500 is out of the system 100 (for example, not in use), the hot surfaces must not be exposed. Additionally, delivery of the liquid formulation (or drug) cannot begin until the cartridge 500 can be fully installed into the cartridge receiver 400. In addition, the system 100 is preferably designed and configured such that the cartridge 500 cannot be removed while the liquid formulation (or drug) is being heated and delivered to a patient.

In accordance with an exemplary embodiment, a lock snap feature 638 on each of the covers 620, 630 (FIGS. 12, 15A) can help prevent the user from moving the active part of the cartridge 600 forward within the covers 620, 630 while it is nested on the placement pins 418 in the cartridge receiver 400 of the aerosol delivery unit 130. In addition, protective arms or latches 636 (FIG. 15A) can keep the active part of the cartridge 600 within the covers 620, 630, for example, in the event the disposable cartridge assembly 500 is dropped. A protective feature 639 (FIG. 12) on the front of the protective arms 636 as disclosed herein can also help prevent the user from being able to place their finger on the tip of the capillary tube 650. A molded spring 632 (see FIG. 11) biases the covers 620, 630, and the device identifying card 670 (see FIG. 12) toward the cartridge identification PCB assembly 422 (FIG. 7A). The cartridge 500 can include a protective sleeve 648 for tubing 691 into a proximal end 653 of the capillary tube 650 (FIG. 12).

In accordance with an exemplary embodiment, as shown in FIG. 12, the right and left plastic covers 620, 630 of the disposable cartridge assembly (or cartridge) 500 are configured to enclose the hot parts, for example, the capillary tube 650 and the susceptor 660. The covers 620, 630 can be locked into place, for example, with internal latches 636 (FIGS. 15A and 15B). When the cartridge 500 is inserted into the starting position in the cartridge receiver 400 of aerosol delivery unit 130, the latches 636 are retracted by placement pins 418 (not shown), thereby, releasing the active part of the cartridge 600. Once the active part of the cartridge 600 is moved into the inductor 450, the covers 620, 630 are disengaged and left in the initial position at the proximal end 131 of the aerosol delivery unit 130. The user can then slide the active part of the cartridge 600 forward by the handle 610, engaging the susceptor 660 and the capillary tube 650 into the engaged position with the transition adapter 700. In accordance with an exemplary embodiment, sensors 408A, 408B can determine if the susceptor 660 and transition adaptor 700 are in the engaged position before drug delivery will begin. In accordance with an exemplary embodiment, the covers 620, 630 are preferably plastic, for example, a molded plastic.

In accordance with an exemplary embodiment, rather than being an oval or rectangular shaped handle, the handle 610 can be any suitable shape, which can easily insert and remove the disposable cartridge assembly 500 from the cartridge receiver 400.

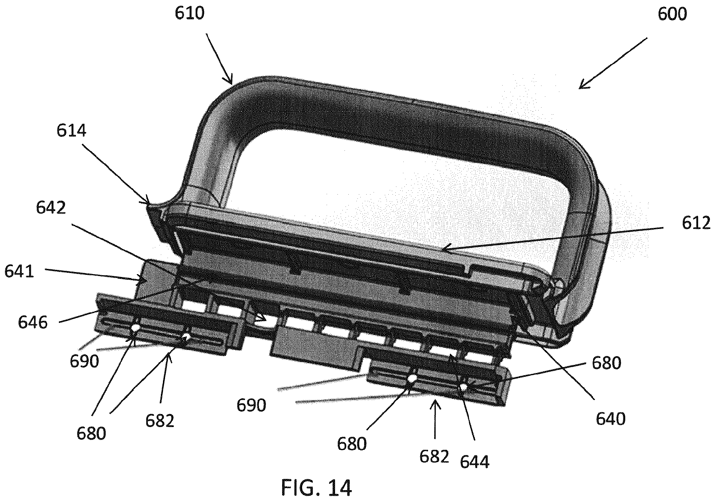

FIG. 13 is a perspective view of a portion of the cartridge 500 in accordance with an exemplary embodiment. This portion is designated as the active part of the cartridge 600 and includes a handle 610, a flat portion of the handle 612, an insulator 640, a susceptor 660, a capillary tube 650 with a distal capillary tip 651 which will be connected to a transition adapter 700 (FIG. 18) and a proximal end of the capillary 653 connected to a tubing connector 693 and a tubing 691 (FIGS. 11 and 12) for delivery of liquid drug to the capillary tube 650. In accordance with an exemplary embodiment, the active part of the cartridge 600 can have a sensor flag 641 located preferably on the distal end of the insulator, which blocks the light so that the optical sensor 408B (FIG. 7A) is engaged to show that the connection between the susceptor 660 and the transition adapter 700 (FIGS. 9A and 9B) has been made. In addition, the insulator 640 can include a sliding guide 646 (FIG. 14) configured to allow the active part of the cartridge 600 to slide in the active alignment groove 420 (FIG. 7A) as the active part of the cartridge 600 moves forward into the inductor 450.

As shown in FIGS. 13 and 14, the insulator 640 can include one or more flanged or angular surfaces 682 with a plurality of wicking ports 680 for attaching the insulator 640 to the susceptor 660 with an epoxy. The epoxy can be conductive or non-conductive. In accordance with an exemplary embodiment, the handle 610 is attached to the flat portion 612 of the handle 610 and then to the insulator 640 using a heat staking technique. Alternatively, these parts can be made as one or two parts by molding or other known methods. In accordance with an exemplary embodiment, a plurality of cut-outs 644 are located between the insulator 640 and the one or more flanges or angled surfaces 682, or an upper surface 661, or the one or more flanges or angled surfaces 682 and an upper surface 661, of the susceptor 660. The plurality of cut-outs 644 can be configured to help dissipated the heat transfer from the susceptor 660 to the handle 610.

FIG. 14 is another perspective view of a portion of the disposable cartridge assembly 500 in accordance with an exemplary embodiment. As shown in FIG. 14, the insulator 640 includes one or more wicking paths 690 on the inside of the insulator 640 to help spread the conductive epoxy along the susceptor 660 (not shown). In addition, the insulator 640 can include a pin lock slot or notch 642 on each side. In accordance with an exemplary embodiment, the placement pins 418 located in the distal end of the cartridge receiver 400 have an additional function of releasing the covers 620, 630 when the cartridge 500 is placed in the initial position. It is also possible to use a separate set of pins or comparable items to unlock and release the covers 620, 630. Each cover 620, 630 has a placement pin access slot 635 to allow the placement pin 418 to go in when the cartridge 500 is placed into the initial position. The placement pin and the locking pin 418 can be referred to interchangeably.

FIGS. 15A-15D are perspective views of a cover 620, 630 of the disposable cartridge assembly 500 in accordance with an exemplary embodiment. In accordance with an exemplary embodiment, each of the covers 620, 630 can include a plurality of heat dissipating features (for example, fins) 634. At least one of the covers 620, 630 can have one or more, preferably four, snap features 631 which would snap into a corresponding indentation or opening in the opposite cover such that both covers would come together to enclose the active part of the cartridge 600 (FIG. 13). In addition, at least one of the covers 620, 630 can have one or more, preferably two, overlapping snap features 633 to further ensure the integrity of the enclosure of the active part of the cartridge within the covers. In addition, one of the covers 620, 630, can include latches 636 configured to engage the insulator 640 by catching the pin notch lock 642 (FIG. 14), and which can help prevent the user from moving the active part of the cartridge 600 forward.

In accordance with an exemplary embodiment, the placement pins 418 in the cartridge receiver 400 of the aerosol delivery unit 130 release (or unlock) the active part of the cartridge 600 from the covers 620, 630 once the disposable cartridge 500 has been nested or correctly positioned within the cartridge receiver 400. When the cartridge 500 is placed on the placement pins 418 (FIG. 7B), the placement pins 418 go through the placement pin access slot 635 and disengage the covers 620, 630 from the active part of the cartridge 600 by pushing away the latches 636 and release the catch of the latches from the pin lock notches 642. Once the active part of the cartridge 600 is released, the covers 620, 630 continue to stay together. When the active part of the cartridge 600 is returned into the covers 620, 630, the cartridge 500 can be lifted off the placement pins. The placement pins 418 then release the latches 636 and the lock snap feature 638. In accordance with an exemplary embodiment, at least one of the covers 620, 630 contains a data card retainer 671 to hold the data card 670. One or more alignment pins 637 is located below the inner edge 643 of one or each of the cover 620, 630. The alignment pin 637 works with the sliding guide 646 (FIG. 14) on the insulator 640 and helps to guide the active part of the cartridge 600 into the alignment groove 420 (FIG. 9A).

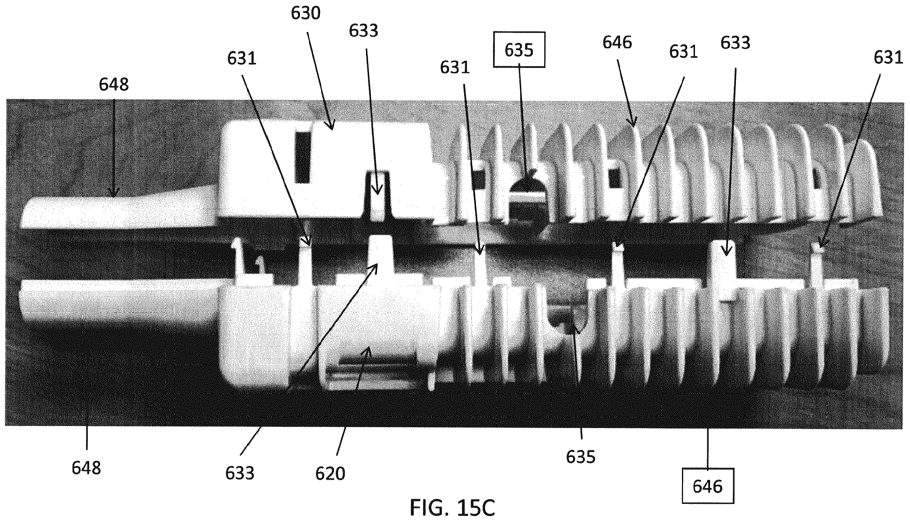

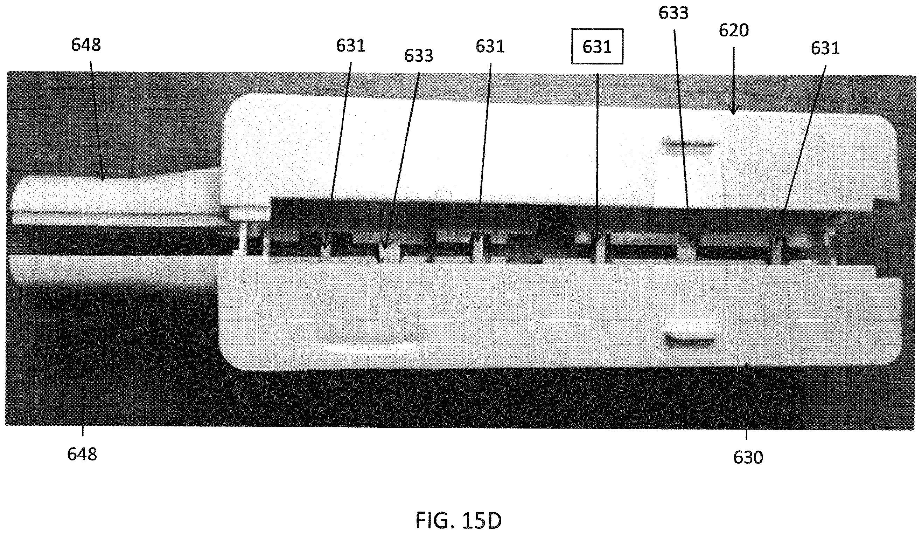

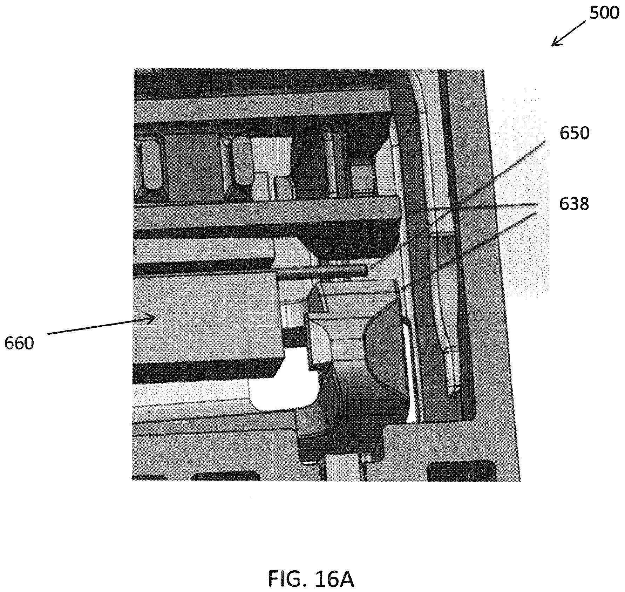

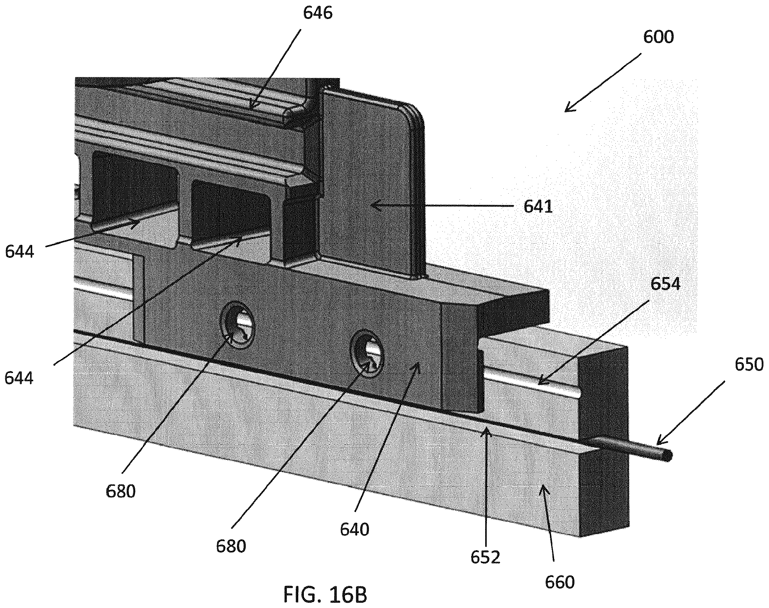

FIG. 16A is a perspective view of an inner portion of the disposable cartridge assembly 500 in accordance with an exemplary embodiment. FIG. 16B is a view of a portion of the susceptor 660, the capillary 650 and the insulator 640. As shown in FIG. 16A, both of the lock snap features 638 (see also FIGS. 12 and 15) on the right and left covers 620, 630, extend out in front (that is, distal end) of the capillary tube 650 to prevent the user from, for example, poking a finger into the area of the tip 651 of the capillary tube 650 and getting burned. For example, the protective arms 638 provide this protection further enhanced by the addition of the protective feature 639 (FIG. 15). The lower arm 638 can wrap around the front end or portion (that is, distal end) of the susceptor 660 in the normal position, for example, the upper protective arm 638 can be positioned above the capillary tube 650, and the lower protective arm 636 can be located alongside the capillary tube 650.

FIG. 16B taken together with FIG. 14 show the connection of the susceptor 660 with the insulator 640. The susceptor 660 has a first groove 652 for the placement of the capillary 650. Preferably, the capillary 650 can be secured within the first groove by the application of conductive epoxy. The susceptor 660 has a second groove 654 which is configured to align with the wicking paths 690 of the insulator 640 in (FIG. 14) forming a hollow structure. When the epoxy is applied through the wicking ports 680, the epoxy spreads inside the hollow structure. When the epoxy is solidified, it forms a lock holding the susceptor 660 and the insulator 640 together. The epoxy does not have to be an adhesive.

In accordance with an exemplary embodiment, since a replaceable cartridge 500 can be a critical component in a drug delivery system, it is preferable that a new cartridge 500 is used for every treatment and disposed of afterwards. In addition, the susceptor 660 within the cartridge 500 can reach up to 300°C or higher while administering the aerosol. Upon completion of the treatment, the cartridge 500 may need to be removed immediately from the aerosol delivery unit 130, before the cartridge 500 has had time to cool to a safe temperature. Therefore, as disclosed herein, measures need to be taken to prevent exposure to the user of the capillary tube 650 and the susceptor 660 of the cartridge 500 immediately following use.

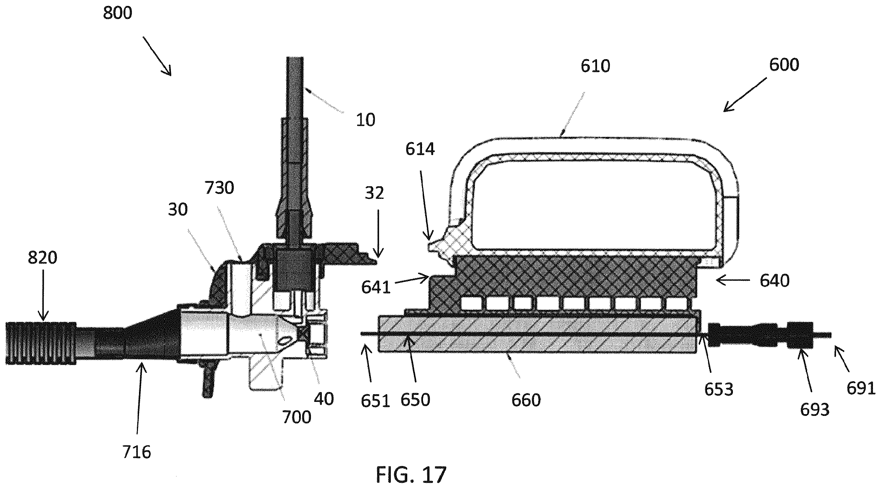

FIG. 17 is a cross-sectional view of the active part of the cartridge 600, the transition adapter 700, and a portion of the tube set assembly 800 in accordance with an exemplary embodiment. As shown in FIG. 17, a disposable tube set assembly 800 can include the transition adapter 700, a carrier gas tubing 10, a transition adapter cover 30, a capillary seal 40, a transition adaptor fitting 716 which connects the transition adaptor 700 with the first tubing 820. The disposable tube set assembly 800 is further described in detail below and shown in FIG. 22.

A tubing 691 for delivery of liquid drug to the capillary tube (FIG. 17) preferably includes a high pressure tubing, which extends from the syringe pump unit 140 (FIG 1) to a proximal end of the cartridge 500. The high pressure tubing assembly can include the tubing connection 693, which is configured to engage with the proximal end 653 of the capillary tube 650.

In accordance with an exemplary embodiment, the transition adapter 700 installs into a transition adapter cavity 414 (FIG. 6) in the cartridge receiver 400. When the active part of the cartridge 600 slides forward to engage with the transition adapter 700 and the transition adaptor cover 30, the front edge of the handle 614 of the active part of the cartridge 600 captures the cover edge 32 of the cover of the transition adapter 32 and thereby engages the transition adapter 700 (FIG. 17). In accordance with an exemplary embodiment, one or more sensors 408A (FIG. 7A) detects the position of the active part of the cartridge 600 and the presence of the transition adapter 700 such that the system 100 will not initiate treatment if both the active part of the cartridge 600 and the transition adapter 700 are not detected. Once initiated, for example, in accordance with an exemplary embodiment, the lock 458 backs up the spring loaded cartridge latch assembly 430 to prevent the cartridge 500 from being removed.

FIG. 18 is a perspective view of the transition adapter 700 in accordance with an exemplary embodiment. As shown in FIG. 18, the transition adapter 700 includes a housing 710 having an air admixing port 720, a temperature probe port 730, an input port 750, and an exit port 760. In addition, the transition adapter 700 can include a flange or extension portion 702, which is configured to be received within a distal end of the aerosol delivery unit 130. In addition, the transition adapter 700 has a flag 704 which works together with the light sensor 408A (FIG. 7A). When the transition adapter 700 is installed into the transition adapter cavity 414 on the aerosolizing device's cartridge receiver 400, the flag 704 enters the placement groove 426 (see FIG. 6) which signals the proper placement of the transition adaptor 700 by the light sensor 408A (FIG. 7A).

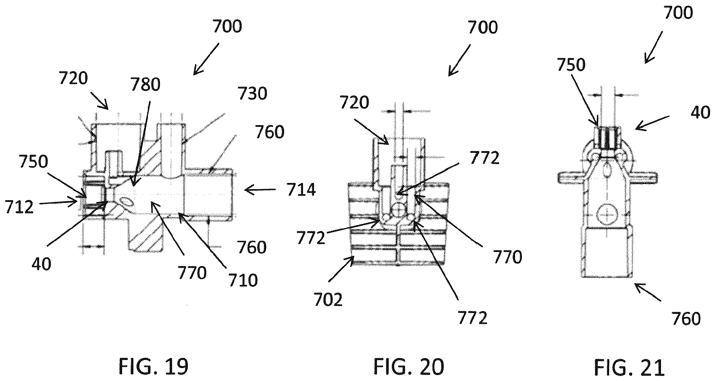

FIGS. 19-21 are cross-sectional views of the transition adapter 700 in accordance with an exemplary embodiment. As shown in FIGS. 18-21, the transition adapter 700 includes a housing 710 having a proximal end 712 and a distal end 714. The proximal end 712 has an aerosol passage or entry port 750 for receiving an aerosol produced by the heated capillary tube 650 of the active part of the cartridge 600. The aerosol passage or entry port 750 preferably includes a capillary seal 40, which contains a connection to a distal tip of the capillary 651. The aerosol enters into an inner cavity 770 (FIG. 19) within the transition adapter 700 through the aerosol passage 750 where the aerosol is at least partially encircled and carried forward by parallel streams of carrier gas, which are originated from a source of gas or ventilator (not shown) and introduced into the transition adapter through at least one gas entry port 720, or alternatively, a plurality of gas entry ports to form an entrained aerosol, which is a combination of the aerosol and the carrier gas. The carrier gas is preferably a non-humidified gas.

As shown in FIGS. 18-21, the aerosol passage 750 has a capillary seal 40, which receives the distal end of the capillary tube 651, which is positioned within a cavity on the proximal end 712 of the housing 710. The housing 710 preferably includes a generally cylindrical proximal portion 712, a cylindrical distal portion 714, and a carrier gas connection port 720 extending perpendicular to the proximal end 712 and configured to receive a carrier gas line 10 (FIG. 17), which transports a stream of carrier gas from the gas source or a ventilator to the transition adapter 700.

FIG. 19 is a side view of the transition adapter 700 as shown in FIG. 18 in accordance with an exemplary embodiment. As shown in FIG. 19, the housing 710 of the transition adapter 700 has a cylindrical proximal portion and a cylindrical distal portion, which extend from the proximal end to the distal end of the housing 710. In accordance with an exemplary embodiment, an outer diameter of the cylindrical proximal portion is smaller than an outer diameter of the cylindrical distal portion. The housing 710 of the transition adapter 700 includes a cylindrical body, which includes a carrier gas connection port 720 for receiving the carrier gas via a carrier gas line from a ventilator or a gas source. The carrier gas connection port 720 has a cylindrical cross-section, which is in communication with a plurality of gas entry ports and a plurality of corresponding gas exit ports 772 via a passage. Each of the gas exit ports 772 delivers a stream of carrier gas to the inner cavity 770 of the transition adapter 700. A temperature probe port 730 is located on an upper portion of the transition adapter 700 and arranged between the carrier gas connection port 720 and a distal end 714 of the transition adapter 700. The temperature probe port 730 is located at the top of the transition adapter's aerosol and carrier gas mixing chamber and can provide access to a temperature probe 160 (FIGS. 1-3A) to assess the temperature of the entrained aerosol as an indicator of the aerosol delivery system operation.

In accordance with another exemplary embodiment as shown in FIGS. 19-21, the source of gas can be introduced into the inner cavity 770 via a single gas entry port and a single gas passage. In accordance with an exemplary embodiment, rather than multiple or a plurality of passages or conduits for introducing the gas stream into cavity 770, the separation of gas streams into the inner cavity 770 can be performed through a plurality of openings or exit ports along the conical section 780. In accordance with an exemplary embodiment, the transition adapter 700 can be configured as shown in commonly-owned U.S. Patent Publication No. 2014/0053831 A1.

In accordance with an exemplary embodiment, the transition adapter 700 installs into the aerosol output pocket 410 on the cartridge receiver 400 (FIG. 6) located at the distal end 133 of the aerosol delivery unit. The cartridge 500 with the attached tubing for delivery of liquid drug to the capillary 691 is placed in the cartridge receiver 400 and slides forward to engage with the transition adapter 700 arranged on the distal side 133 of the cartridge receiver 400 of the aerosol delivery unit 130. The susceptor 660 within the cartridge 500 can be self-aligning to the transition adapter 700, as the transition adapter 700 has some limited freedom to move about during engagement. In accordance with an exemplary embodiment, the handle 610 of the active part of the cartridge 600 can capture or engage a cover of the transition adapter 700 and thereby captures the transition adapter 700. As set forth above, one or more sensors 408A and 408B monitor the position of the active part of the active part of the cartridge 600 and presence of the transition adapter 700 in the cartridge receiver 400. In accordance with an exemplary embodiment, the system 100 will not initiate treatment if both the active part of the cartridge 600 and the aerosol transition adapter 700 are not detected. Once initiated, an electronically controlled interlock 458 closes to prevent the active part of the cartridge 600 from being removed.

FIG. 22 is a cross-sectional view of a tube set assembly 800 in accordance with an exemplary embodiment. A carrier gas tube 10 is configured to deliver a carrier gas from a source to the carrier gas entry port 720 of the transition adaptor 700. As shown in FIG. 22, a disposable aerosol tube set 800 includes the carrier gas tube 10, the transition adapter 700, a transition adapter cover 30, and a capillary seal 40 (FIG. 17), a first piece of tubing 820, the transition adaptor fitting 716 which connects the transition adaptor 700 with the first tubing 820, a condensation trap 830 and a second piece of tubing 840. The carrier gas tube 10 delivers the carrier gas to the transition adaptor 700 where the carrier gas meets with the aerosol delivered from the capillary (not shown) to form an entrained aerosol. The entrained aerosol enters the first piece of tubing 820, passes by the condensation trap 830, and then enters into the second piece of tubing 840, which can be connected to a patient interface (not shown). The disposable aerosol tube set 800 can further include a tee adapter 850 for a temperature probe connected to the second tubing 840, a third tubing 860 and an elbow connector 870 for connecting to a patient interface (not shown).

In accordance with an exemplary embodiment, both tubing 691 together with the tubing connector 693 and the tube set 800 are preferably replaceable. For example, in accordance with an exemplary embodiment, the tube set 800 can be used once per patient, while the tubing 691 (FIG. 17) can be replaced with each dose. Therefore, the tubing 691 is changed with greater frequency and needs to be removable without upsetting the tube set 800, which drives the requirement that the tube set assembly 800 be installed first, before the tubing 691. Additionally, during treatment, neither the tubing 691 nor the tube set 800 can be removed.

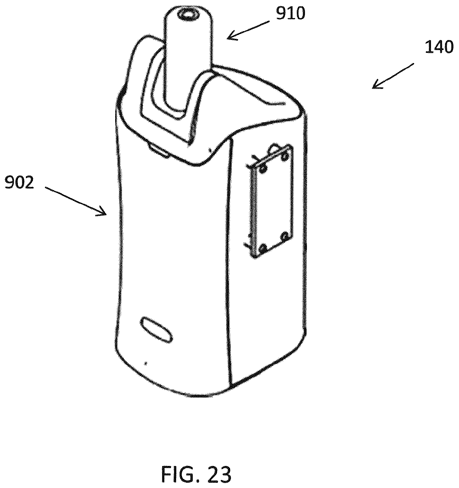

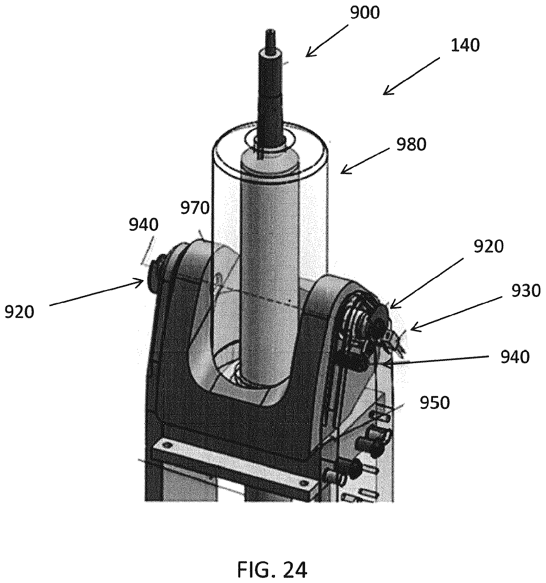

FIG. 23 is a perspective view of the syringe pump unit 140 in accordance with an exemplary embodiment. As shown in FIG. 23, the syringe pump unit 140 can include a housing 902, which is configured such that an operator can load, for example, a disposable syringe 900 (FIG. 24) into the syringe holder 910. The disposable syringe can hold from 20 ml to 200 ml of liquid drug. In accordance with an exemplary embodiment, the syringe holder 910 pivots on a hinge to expose the opening on the bottom of the syringe holder 910. In accordance with an exemplary embodiment, the syringe holder 910 is preferably a clear plastic. The syringe 900 is aligned and inserted into the syringe holder 910. Then, the syringe holder 910 is pivoted back to its original position. In accordance with an exemplary embodiment, an indicator light (not shown), for example, one or more green indicator lights can convey to the operator that the syringe holder 910 is closed and ready for use.

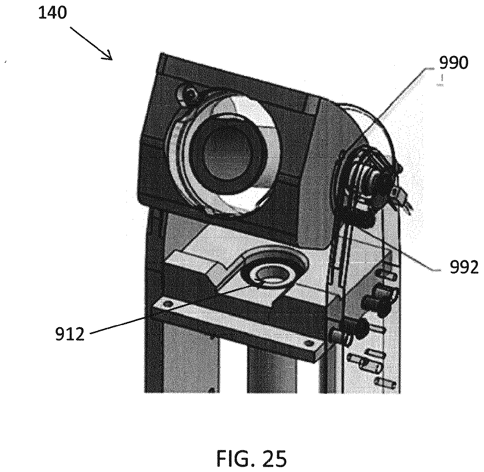

FIG. 24 is a perspective view of the syringe pump unit 140 in an operational state accordance with an exemplary embodiment. As shown in FIG. 24, the syringe pump unit 140 can include an approach for loading and supporting a syringe 900 in a syringe pump 910, which is especially suited for a high pressure syringe pump. In accordance with an exemplary embodiment, a plunger 912 (FIG. 25) presses on the piston (not shown) in the syringe 900, pressurizing the fluid in the syringe 900, which creates a net force coaxial with the syringe 900. In accordance with an exemplary embodiment, a structure, such as the syringe pump holder 910 is needed to hold the syringe 900 in place when the force is applied. In addition, the syringe pump holder 910 should also allow the syringe 900 to be inserted into the pump and removed from the pump 140 relatively easily.

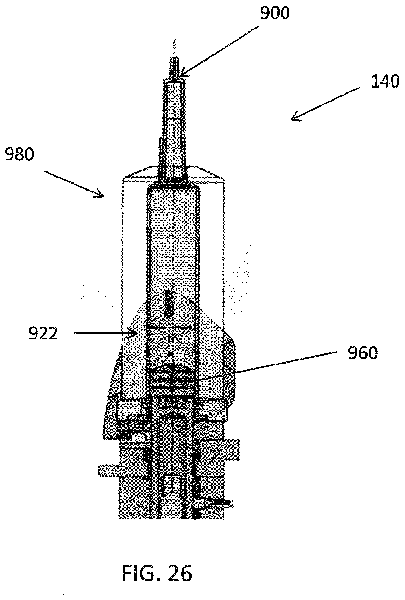

As shown in FIG. 24, the syringe pump unit 140 includes the syringe 900, the pressure jacket holder 970, sensor disks 920, a sensor 930, a pivot axis 940, a stop feature 950, a plunger 960 (FIG. 25), a pressure jacket holder 970, and a pressure jacket 980. The syringe 900 is supported in the pressure jacket 980, which is attached to a pressure jacket holder 970. The pressure jacket holder 970 is mounted to the syringe pump structure on pins 922 (FIG. 26), allowing the pressure jacket holder 970 to rotate as shown in FIG. 25 to allow loading and unloading of the syringe 900. In accordance with an exemplary embodiment, a ball spring plunger (not shown) and detents 990, 992 help the pressure jacket holder 970 stay in the positions needed for loading and operation.

As shown in FIG. 25, detent 990 is for a closed position, and detent 992 is for an open position. In addition, the sensor disks 920 can be mounted to the rotating pins 922, which allows the sensors on the syringe pump structure to detect a position of the pressure jacket holder 970 (FIG. 24) to prevent the plunger 960 (FIG. 26) from advancing before the pressure jacket holder 970 is in the operational position.

FIG. 26 is a perspective view of the syringe pump unit 140 in accordance with an exemplary embodiment. In accordance with an exemplary embodiment, a key feature in the design is the way the pressure jacket holder 970 reacts to the forces applied on it as shown in FIG. 26. In accordance with an exemplary embodiment, the pivot pins 922 can be offset from the axis of the syringe so that a moment is created when the syringe 900 is pressurized. The moment is an offset of the direction of the force applied by the pump plunger 960 and the direction of resistance to the force created by the rotational stop 950. The rotation caused by the pump plunger 960 is stopped by the rotation al stop 950. In FIG. 26, the pressure jacket 980 would tend to rotate counterclockwise due to this moment. Stop features 950 (FIG. 24) on the pressure jacket 980 maintain the position when the moment is applied. With this arrangement, the pressure jacket holder 970 tends to close when the syringe 900 is pressurized, and no additional latching mechanism is needed to hold the pressure jacket 980 in place.

In accordance with an exemplary embodiment, the syringe 900 can include a liquid formulation. For example, the liquid formulation can include a lung surfactant or any other drug preparation adapted for delivery as an aerosol to an infant's lungs or a medicament to treat Respiratory Distress Syndrome (RDS) in infants or any other disease in children and adults. The liquid formulation can be contained within a dose container, such as, for example, a syringe 900, which can be pre-portioned.

The aerosol delivery system 100 is configured to supply the liquid formulation from the dose container at a constant and continuous rate to the heated capillary tube 650, wherein the liquid formulation is at least partially volatized. Alternatively, the liquid formulation is prepared by reconstituting a solid formulation (for example, freeze-dried pharmaceutical formulation) with an appropriate pharmaceutically acceptable carrier such as, for example, water, buffer, or saline solution and optionally heated. Alternatively, multiple liquid formulations containing different drugs or reservoirs containing auxiliary substances other than drugs, for example, pharmaceutically acceptable carriers together with multiple feeding lines, can be provided as needed.

While various embodiments have been disclosed, it is to be understood that variations and modifications may be resorted to as will be apparent to those skilled in the art. Particularly, the outer shape of the transition adapter can be modified without affecting the inner structure. Such variations and modifications are to be considered within the purview and scope of the claims appended hereto.

## Claims

1. An aerosol delivery system (100), the aerosol delivery system (100) comprising:
an aerosol delivery unit (130), the aerosol delivery unit (130) comprising:
a cartridge receiver (400), which is configured to receive a cartridge assembly (500); and
an inductor (450) configured to receive the cartridge assembly (500) and heat a liquid formulation within a capillary tube (650) to produce an aerosol by induction heating; and
a pump (140), the pump (140) comprising:
a container holder (910) configured to receive a container (900) containing the liquid formulation, and wherein the container holder (910) is configured to pivot about an axis to expose an opening on a bottom of the container holder (910);
a plunger (960) for applying a force to push the liquid formulation out of a distal end of the container (910) into a proximal end (653) of the capillary tube (650); and
a pressure jacket (980) configured to support the container (900) within the container holder (910).

2. The aerosol delivery system (100) of claim 1, wherein the container (900) is a syringe.

3. The aerosol delivery system (100) of claim 1 or 2, comprising:
an operator's interface (110), the operator's interface (110) having a display for inputting instructions for a delivery of the aerosol to a patient, or treatment status of the patient, or delivery of the aerosol to a patient and treatment status of the patient; and
a power control unit (120), the power control unit (120) configured to supply a source of power to at least one of the aerosol delivery unit (130), the pump (140), and the operator's interface (110).

4. The aerosol delivery system (100) of claim 3, comprising:
a pole or wheel cart (170) configured to receive one or more of the aerosol delivery unit (130), the pump (140), the operator's interface (110), and the power control unit (120).

5. The aerosol delivery system (100) of any one of claims 1 to 4, further comprising:
a disposable aerosol tube set (800), which includes a transition adapter (700); and
a temperature probe (160), the temperature probe (160) configured to monitor a temperature of the aerosol delivery unit (130) to determine if the aerosol delivery unit (130) is operating within a working temperature range of from approximately 100°C to 300°C.

6. The aerosol delivery system (100) of any one of claims 1 to 5, comprising:
the cartridge assembly (500) configured to be placed within the cartridge receiver (400) of the aerosol delivery unit (130), the cartridge assembly (500) including a pair of displaceable covers (620, 630) and an active part of the cartridge (600), the active part of the cartridge (600) including a susceptor (660), a capillary tube (650) partially surrounded by the susceptor (660), an insulator (640), and a handle (610), the insulator (640) arranged between the susceptor (660) and the handle (610) and wherein the pair of displaceable covers (620, 630) is configured to surround the insulator (640), the capillary tube (650) and the susceptor (660).

7. The aerosol delivery system of claim 6, wherein each of the pair of displaceable covers (620, 630) includes one or both of:
heat dissipating fins (634), the heat dissipating fins (634) configured to dissipate heat and create a space between a user and an exposed heated capillary tip (651) and the susceptor (660); and
a protective arm (638), the protective arm (638) configured to surround a distal end of the susceptor (660) and a distal end (651) of the capillary tube (650).

8. The aerosol delivery system (100) of claim 6 or 7, comprising:
a pair of placement pins (418) arranged within the cartridge receiver (400) and configured to receive the cartridge assembly (500) and to separate the pair of displaceable covers (620, 630) on the cartridge assembly (500) from the active part of the cartridge (600).

9. The aerosol delivery system (100) of any one of claims 1 to 8, comprising:
a cartridge identification assembly (422) arranged within the cartridge receiver (400), the cartridge identification assembly (422) configured to identify a data card (670) on the cartridge assembly (500).

10. The aerosol delivery system (100) of any one of claims 1 to 9, wherein the aerosol delivery unit (130) includes a cartridge lock (458), the cartridge lock (458) configured to lock the cartridge assembly (500) within the cartridge receiver (400).

11. The aerosol delivery system (100) of any one of claims 1 to 10, wherein the inductor (450) includes an induction coil (462) and wherein the induction coil (462) comprises a wire (461) wound around one or more graphite cores (464).

12. The aerosol delivery system (100) of any one of claims 1 to 11, comprising:
an aerosol transition adapter (700) for receiving the aerosol and combining the aerosol with a carrier gas, wherein the aerosol transition adapter (700) includes a transition adapter flag (704) adapted to be detected by a sensor (408A) to signal when the aerosol transition adapter (700) is being placed within the cartridge receiver (400).

13. The aerosol delivery system of claim 12, wherein the aerosol transition adapter comprises:
a housing (710) having a proximal end (712) and a distal end (714), the proximal end (712) having an aerosol passage (750) for receiving the aerosol from the capillary tube (650) and the distal end (714) having an exit port (760), the housing (710) having a length between the distal end (714) and the proximal end (712);
a carrier gas connection port (720) for receiving the carrier gas from a gas source, which is in communication with a plurality of carrier gas exit ports (772), the plurality of carrier gas exit ports (772) are arranged adjacent to the aerosol passage (750) in a pattern that partially encircles the flow of aerosol;
an inner cavity (770), which is adapted to receive the aerosol from the aerosol passage (750) and the carrier gas from the plurality of carrier gas exit ports (772) and to direct streams of carrier gas to at least partially encircle and flow in parallel with a main direction of a flow of the aerosol along the length of the housing (710) toward the exit port (760); and
the exit port (760) on the distal end (714) of the housing (710) for delivering the aerosol to a patient in need of an aerosolized active agent.

14. The aerosol delivery system of claim 13, wherein the inner cavity (770) has a proximal portion having a conical inner wall (780), which expands outward towards the distal end (714) of the housing (710), and a distal portion having a tapered inner diameter; and
wherein the carrier gas connection port (720) for receiving the carrier gas from the gas source includes at least one gas entry port for receiving the carrier gas, the at least one gas entry port directing a stream of carrier gas to one or more gas exit ports (772).

15. The aerosol delivery system of any one of claims 12 to 14, wherein the cartridge receiver (400) includes:
an active alignment groove (420) configured to guide the active part of the cartridge assembly (600) into the inductor (650), a transition adapter cavity (414), and a transition adapter placement groove (426), which connects with the transition adapter flag (704) on the aerosol transition adapter (700), such that when the aerosol transition adapter (700) is inserted into the transition adapter placement groove (426), the transition adapter flag (704) is detected by the sensor (408A), which signals that the aerosol transition adapter (700) has been placed in the cartridge receiver (400).

16. The aerosol delivery system (100) of any one of claims 6 to 15, wherein the aerosol delivery unit (130) includes one or more sensors (408A, 408B), the one or more sensors (408A, 408B) configured to detect a presence of the cartridge assembly (500), or the active part of the cartridge (600), or the cartridge assembly (500) and the active part of the cartridge (600), within the cartridge receiver (400), a presence of a transition adapter (700) within the cartridge receiver (400), and an engagement of a capillary tip (651) of the capillary tube (650) with the transition adapter (700) within the cartridge receiver (400) of the aerosol delivery unit (130) upon moving the active part of the cartridge (600) from a proximal end of the cartridge receiver (400) to a distal end of the cartridge receiver (400).

## Patentansprüche

1. Aerosolabgabesystem (100), das Aerosolabgabesystem (100) umfassend:
eine Aerosolabgabeeinheit (130), wobei die Aerosolabgabeeinheit (130) aufweist:
eine zur Aufnahme einer Patronenbaugruppe (500) ausgelegte Patronenaufnahme (400); und
einen zur Aufnahme der Patronenbaugruppe (500) und zum Erwärmen einer flüssigen Formulierung in einem Kapillarrohr (650) ausgelegten Induktor (450), um ein Aerosol durch Induktionserwärmung zu erzeugen; und
eine Pumpe (140), die Pumpe (140) umfassend:
einen Behälterhalter (910), der zur Aufnahme eines die flüssige Formulierung enthaltenden Behälters (900) ausgelegt ist, und wobei der Behälterhalter (910) zum Schwenken um eine Achse ausgelegt ist, um eine Öffnung an einem Boden des Behälterhalters (910) freizulegen;
einen Kolben (960) zum Aufbringen einer Kraft, um die flüssige Formulierung aus einem distalen Ende des Behälters (910) in ein proximales Ende (653) des Kapillarrohrs (650) zu drücken; und
einen Druckmantel (980), der zum Tragen des Behälters (900) innerhalb des Behälterhalters (910) ausgelegt ist.

2. Aerosolabgabesystem (100) nach Anspruch 1, wobei der Behälter (900) eine Spritze ist.

3. Aerosolabgabesystem (100) nach Anspruch 1 oder 2, umfassend:
eine Bedieneroberfläche (110), wobei die Bedieneroberfläche (110) eine Anzeige zur Eingabe von Anweisungen für die Abgabe des Aerosols an einen Patienten oder den Behandlungsstatus des Patienten oder die Abgabe des Aerosols an einen Patienten und den Behandlungsstatus des Patienten aufweist; und
eine Energiesteuereinheit (120), wobei die Energiesteuereinheit (120) zum Vorsehen einer Energiequelle für wenigstens eine der Aerosolabgabeeinheiten (130), die Pumpe (140) und die Bedieneroberfläche (110) ausgelegt ist.

4. Aerosolabgabesystem (100) nach Anspruch 3, umfassend:
einen Ständer oder einen Rollwagen (170), ausgelegt zur Aufnahme einer oder mehrerer der Aerosolabgabeeinheit (130), der Pumpe (140), der Bedieneroberfläche (110) und der Energiesteuereinheit (120).

5. Aerosolabgabesystem (100) nach einem der Ansprüche 1 bis 4, ferner umfassend:
einen Einweg-Aerosolschlauchsatz (800), der einen Übergangsadapter (700) beinhaltet; und
eine Temperatursonde (160), wobei die Temperatursonde (160) zur Überwachung einer Temperatur der Aerosolabgabeeinheit (130) ausgelegt ist, um zu ermitteln, ob die Aerosolabgabeeinheit (130) innerhalb eines Arbeitstemperaturbereichs von ungefähr 100 °C bis 300 °C arbeitet.

6. Aerosolabgabesystem (100) nach einem der Ansprüche 1 bis 5, umfassend:
die zum Platzieren in der Patronenaufnahme (400) der Aerosolabgabeeinheit (130) ausgelegte Patronenbaugruppe (500), wobei die Patronenbaugruppe (500) ein Paar von verschiebbaren Abdeckungen (620, 630) und einen aktiven Teil der Patrone (600) beinhaltet, wobei der aktive Teil der Patrone (600) einen Suszeptor (660), ein teilweise von dem Suszeptor (660) umgebenes Kapillarrohr (650), einen Isolator (640) und einen Griff (610) beinhaltet, wobei der Isolator (640) zwischen dem Suszeptor (660) und dem Griff (610) angeordnet ist und wobei das Paar von verschiebbaren Abdeckungen (620, 630) zum Umgeben des Isolators (640), des Kapillarrohrs (650) und des Suszeptors (660) ausgelegt ist.

7. Aerosolabgabesystem nach Anspruch 6, wobei jedes Paar verschiebbarer Abdeckungen (620, 630) eines oder beides enthält von:
wärmeableitenden Rippen (634), wobei die wärmeableitenden Rippen (634) zum Ableiten von Wärme und zur Schaffung eines Raums zwischen einem Benutzer und einer freiliegenden erwärmten Kapillarspitze (651) und dem Suszeptor (660) ausgelegt sind; und
einen Schutzarm (638), wobei der Schutzarm (638) ausgelegt ist, um ein distales Ende des Suszeptors (660) und ein distales Ende (651) des Kapillarrohrs (650) zu umgeben.

8. Aerosolabgabesystem (100) nach Anspruch 6 oder 7, umfassend:
ein Paar von Platzierungsstiften (418), die innerhalb der Patronenaufnahme (400) angeordnet und zur Aufnahme der Patronenbaugruppe (500) und zur Trennung des Paares von verschiebbaren Abdeckungen (620, 630) auf der Patronenbaugruppe (500) von dem aktiven Teil der Patrone (600) ausgelegt sind.

9. Aerosolabgabesystem (100) nach einem der Ansprüche 1 bis 8, umfassend:
eine in der Patronenaufnahme (400) angeordnete Patronenidentifikationsbaugruppe (422), wobei die Patronenidentifikationsbaugruppe (422) zum Identifizieren einer Datenkarte (670) auf der Patronenbaugruppe (500) ausgelegt ist.

10. Aerosolabgabesystem (100) nach einem der Ansprüche 1 bis 9, wobei die Aerosolabgabeeinheit (130) eine Patronenverriegelung (458) beinhaltet, wobei die Patronenverriegelung (458) zum Verriegeln der Patronenbaugruppe (500) innerhalb der Patronenaufnahme (400) ausgelegt ist.

11. Aerosolabgabesystem (100) nach einem der Ansprüche 1 bis 10, wobei der Induktor (450) eine Induktionsspule (462) beinhaltet und wobei die Induktionsspule (462) einen Draht (461) umfasst, der um einen oder mehrere Graphitkerne (464) gewickelt ist.

12. Aerosolabgabesystem (100) nach einem der Ansprüche 1 bis 11, umfassend:
einen Aerosolübergangsadapter (700) zum Aufnehmen des Aerosols und Kombinieren des Aerosols mit einem Trägergas, wobei der Aerosolübergangsadapter (700) eine Übergangsadaptermarkierung (704) beinhaltet, die zum Detektieren durch einen Sensor (408A) angepasst ist, um zu signalisieren, wenn der Aerosolübergangsadapter (700) in die Patronenaufnahme (400) eingesetzt ist.

13. Aerosolabgabesystem nach Anspruch 12, wobei der Aerosolübergangsadapter umfasst:
ein Gehäuse (710), aufweisend ein proximales Ende (712) und ein distales Ende (714), wobei das proximale Ende (712) einen Aerosolkanal (750) zur Aufnahme des Aerosols aus dem Kapillarrohr (650) aufweist und das distale Ende (714) eine Austrittsöffnung (760) aufweist, wobei das Gehäuse (710) eine Länge zwischen dem distalen Ende (714) und dem proximalen Ende (712) aufweist;
eine Trägergasverbindungsanschluss (720) zur Aufnahme des Trägergases von einer Gasquelle, die mit einer Vielzahl von Trägergasaustrittsanschlüssen (772) verbunden ist, wobei die Vielzahl von Trägergasaustrittsanschlüssen (772) angrenzend an den Aerosolkanal (750) in einem Muster angeordnet sind, das den Aerosolstrom teilweise umschließt;
einen Innenhohlraum (770), der für die Aufnahme des Aerosols aus dem Aerosolkanal (750) und des Trägergases aus der Vielzahl von Trägergasaustrittsanschlüssen (772) und zum Leiten von Trägergasströmen ausgelegt ist, sodass diese wenigstens teilweise eine Hauptströmungsrichtung des Aerosols entlang der Länge des Gehäuses (710) in Richtung des Austrittsanschlusses (760) umschließen und parallel dazu strömen; und
den Austrittsanschluss (760) an dem distalen Ende (714) des Gehäuses (710), um das Aerosol an einen Patienten abzugeben, der einen aerosolierten Wirkstoff benötigt.

14. Aerosolabgabesystem nach Anspruch 13, wobei der Innenhohlraum (770) einen proximalen Abschnitt mit einer konischen Innenwand (780), die sich nach außen zu dem distalen Ende (714) des Gehäuses (710) hin erweitert, und einen distalen Abschnitt mit einem sich verengenden Innendurchmesser aufweist; und
wobei der Trägergasverbindungsanschluss (720) zur Aufnahme des Trägergases von der Gasquelle wenigstens einen Gaseintrittsanschluss zur Aufnahme des Trägergases beinhaltet, wobei der wenigstens eine Gaseintrittsanschluss einen Trägergasstrom zu einem oder mehreren Gasaustrittsanschlüssen (772) leitet.

15. Aerosolabgabesystem nach einem der Ansprüche 12 bis 14, wobei die Patronenaufnahme (400) beinhaltet:
eine aktive Ausrichtungsnut (420), die zum Führen des aktiven Teils der Patronenbaugruppe (600) in den Induktor (650) ausgelegt ist, einen Übergangsadapterhohlraum (414) und eine Übergangsadapterplatzierungsnut (426), die sich mit der Übergangsadaptermarkierung (704) an dem Aerosolübergangsadapter (700) verbindet, sodass, wenn der Aerosolübergangsadapter (700) in die Übergangsadapterplatzierungsnut (426) eingeführt wird, die Übergangsadaptermarkierung (704) von dem Sensor (408A) detektiert wird, der signalisiert, dass der Aerosolübergangsadapter (700) in der Patronenaufnahme (400) platziert wurde.

16. Aerosolabgabesystem (100) nach einem der Ansprüche 6 bis 15, wobei die Aerosolabgabeeinheit (130) einen oder mehrere Sensoren (408A, 408B) beinhaltet, wobei der eine oder die mehreren Sensoren (408A, 408B) zur Detektion eines Vorhandenseins der Patronenbaugruppe (500) oder des aktiven Teils der Patrone (600) oder der Patronenbaugruppe (500) und des aktiven Teils der Patrone (600) innerhalb der Patronenaufnahme (400), des Vorhandenseins eines Übergangsadapters (700) innerhalb der Patronenaufnahme (400) und eines Eingriffs einer Kapillarspitze (651) des Kapillarrohrs (650) mit dem Übergangsadapter (700) innerhalb der Patronenaufnahme (400) der Aerosolabgabeeinheit (130) beim Bewegen des aktiven Teils der Patrone (600) von einem proximalen Ende der Patronenaufnahme (400) zu einem distalen Ende der Patronenaufnahme (400) ausgelegt ist.

## Revendications

1. Système de libération d'aérosol (100), le système de libération d'aérosol (100) comprenant :
une unité de libération d'aérosol (130), l'unité de libération d'aérosol (130) comprenant :
un récepteur de cartouche (400), qui est configuré pour recevoir un ensemble de cartouche (500) ; et
un inducteur (450) configuré pour recevoir l'ensemble de cartouche (500) et chauffer une formulation liquide à l'intérieur d'un tube capillaire (650) pour produire un aérosol par chauffage par induction ; et
une pompe (140), la pompe (140) comprenant :
un support de récipient (910) configuré pour recevoir un récipient (900) contenant la formulation liquide, et dans lequel le support de récipient (910) est configuré pour pivoter autour d'un axe pour exposer une ouverture sur un fond du support de récipient (910) ;
un piston (960) destiné à appliquer une force pour pousser la formulation liquide hors d'une extrémité distale du récipient (910) dans une extrémité proximale (653) du tube capillaire (650) ; et
une gaine de pression (980) configurée pour supporter le récipient (900) à l'intérieur du support de récipient (910).

2. Système de libération d'aérosol (100) selon la revendication 1, dans lequel le récipient (900) est une seringue.

3. Système de libération d'aérosol (100) selon la revendication 1 ou 2, comprenant :
une interface d'opérateur (110), l'interface d'opérateur (110) ayant un affichage destiné à entrer des instructions pour une libération de l'aérosol vers un patient, ou un état de traitement du patient, ou une libération de l'aérosol vers un patient et un état de traitement du patient ; et
une unité de commande de puissance (120), l'unité de commande de puissance (120) étant configurée pour fournir une source de puissance à au moins l'une de l'unité de libération d'aérosol (130), de la pompe (140) et de l'interface d'opérateur (110).

4. Système de libération d'aérosol (100) selon la revendication 3, comprenant :
un chariot à poteau ou à roue (170) configuré pour recevoir une ou plusieurs parmi l'unité de libération d'aérosol (130), la pompe (140), l'interface d'opérateur (110) et l'unité de commande de puissance (120).

5. Système de libération d'aérosol (100) selon l'une quelconque des revendications 1 à 4, comprenant en outre :
un ensemble de tube d'aérosol jetable (800), qui comprend un adaptateur de transition (700) ; et
une sonde de température (160), la sonde de température (160) étant configurée pour surveiller une température de l'unité de libération d'aérosol (130) pour déterminer si l'unité de libération d'aérosol (130) fonctionne dans une plage de température de travail d'environ 100 °C à 300 °C.

6. Système de libération d'aérosol (100) selon l'une quelconque des revendications 1 à 5, comprenant :
l'ensemble de cartouche (500) configuré pour être placé dans le récepteur de cartouche (400) de l'unité de libération d'aérosol (130), l'ensemble de cartouche (500) comportant une paire de couvercles déplaçables (620, 630) et une partie active de la cartouche (600), la partir active de la cartouche (600) comportant un suscepteur (660), un tube capillaire (650) entouré partiellement le suscepteur (660), un isolant (640) et une poignée (610), l'isolant (640) étant disposés sur le suscepteur (660) et la poignée (610), et dans lequel la paire de couvercles déplaçables (620, 630) est configurée pour entourer l'isolant (640), le tube capillaire (650) et le suscepteur (660).

7. Système de libération d'aérosol selon la revendication 6, dans lequel chacun de la paire de couvercles déplaçables (620, 630) comprend l'un ou les deux parmi :
des ailettes de dissipation de chaleur (634), les ailettes de dissipation de chaleur (634) étant configurées pour dissiper la chaleur et créer un espace entre un utilisateur et une pointe capillaire chauffée exposée (651) et le suscepteur (660) ; et
un bras de protection (638), le bras de protection (638) étant configuré pour entourer une extrémité distale du suscepteur (660) et une extrémité distale (651) du tube capillaire (650).

8. Système de libération d'aérosol (100) selon la revendication 6 ou 7, comprenant :
une paire de broches de placement (418) disposées à l'intérieur du récepteur de cartouche (400) et configurées pour recevoir l'ensemble de cartouche (500) et pour séparer la paire de couvercles déplaçables (620, 630) sur l'ensemble de cartouche (500) de la partie active de la cartouche (600).

9. Système de libération d'aérosol (100) selon l'une quelconque des revendications 1 à 8, comprenant :
un ensemble d'identification de cartouche (422) disposé dans le récepteur de cartouche (400), l'ensemble d'identification de cartouche (422) étant configuré pour identifier une carte de données (670) sur l'ensemble de cartouche (500).

10. Système de libération d'aérosol (100) selon l'une quelconque des revendications 1 à 9, dans lequel l'unité de libération d'aérosol (130) comporte un verrou de cartouche (458), le verrou de cartouche (458) étant configuré pour verrouiller l'ensemble de cartouche (500) à l'intérieur du récepteur de cartouche (400).

11. Système de libération d'aérosol (100) selon l'une quelconque des revendications 1 à 10, dans lequel l'inducteur (450) comporte une bobine d'induction (462) et dans lequel la bobine d'induction (462) comprend un fil (461) enroulé autour d'un ou plusieurs noyaux de graphite (464).

12. Système de libération d'aérosol (100) selon l'une quelconque des revendications 1 à 11, comprenant :
un adaptateur de transition d'aérosol (700) destiné à recevoir l'aérosol et à combiner l'aérosol avec un gaz porteur, dans lequel l'adaptateur de transition d'aérosol (700) comprend un indicateur d'adaptateur de transition (704) adapté pour être détecté par un capteur (408A) afin de signaler quand l'adaptateur de transition d'aérosol (700) est placé à l'intérieur du récepteur de cartouche (400) .

13. Système de libération d'aérosol selon la revendication 12, dans lequel l'adaptateur de transition d'aérosol comprend :
un logement (710) ayant une extrémité proximale (712) et une extrémité distale (714), l'extrémité proximale (712) ayant un passage d'aérosol (750) destiné à recevoir l'aérosol du tube capillaire (650) et l'extrémité distale (714) ayant un orifice de sortie (760), le logement (710) ayant une longueur entre l'extrémité distale (714) et l'extrémité proximale (712) ;
un port de connexion de gaz porteur (720) destiné à recevoir un gaz porteur provenant d'une source de gaz, qui est en communication avec une pluralité de ports de sortie de gaz porteur (772), la pluralité de ports de sortie de gaz porteur (772) sont agencés adjacents au passage d'aérosol (750) dans un motif qui encercle partiellement l'écoulement d'aérosol ;
une cavité interne (770), qui est adaptée pour recevoir l'aérosol du passage d'aérosol (750) et du gaz porteur à partir de la pluralité de ports de sortie de gaz porteur (772) et pour diriger des courants de gaz porteur pour encercler au moins partiellement, et s'écouler en parallèle avec, une direction principale d'un écoulement d'aérosol sur la longueur du logement (710) vers le port de sortie (760) ; et
l'orifice de sortie (760) sur l'extrémité distale (714) du logement (710) pour fournir l'aérosol à un patient ayant besoin d'un agent actif en aérosol.

14. Système de libération d'aérosol selon la revendication 13, dans lequel la cavité interne (770) a une partie proximale ayant une paroi interne conique (780), qui s'élargit vers l'extérieur vers l'extrémité distale (714) du logement (710), et une partie distale ayant un diamètre interne conique ; et
dans lequel le port de connexion de gaz porteur (720) pour la réception du gaz porteur provenant de la source de gaz comporte au moins un port d'entrée de gaz pour la réception du gaz porteur, l'au moins un port d'entrée de gaz dirigeant un flux de gaz porteur vers un ou plusieurs ports de sortie de gaz (772).

15. Système de libération d'aérosol selon l'une quelconque des revendications 12 à 14, dans lequel le récepteur de cartouche (400) comporte :
une rainure d'alignement active (420) configurée pour guider la partie active de l'ensemble de cartouche (600) dans l'inducteur (650), une cavité d'adaptateur de transition (414), et une rainure de placement d'adaptateur de transition (426), qui se connecte à l'indicateur d'adaptateur de transition (704) sur l'adaptateur de transition d'aérosol (700), de telle sorte que lorsque l'adaptateur de transition d'aérosol (700) est inséré dans la rainure de placement d'adaptateur de transition (426), l'indicateur d'adaptateur de transition (704) est détecté par le capteur (408A), qui signale que l'adaptateur de transition d'aérosol (700) a été placé dans le récepteur de cartouche (400).

16. Système de libération d'aérosol (100) selon l'une quelconque des revendications 6 à 15, dans lequel l'unité de libération d'aérosol (130) comporte un ou plusieurs capteurs (408A, 408B), les un ou plusieurs capteurs (408A, 408B) étant configurés pour détecter une présence de l'ensemble de cartouche (500), ou la partie active de la cartouche (600), ou l'ensemble de cartouche (500) et la partie active de la cartouche (600), à l'intérieur du récepteur de cartouche (400), une présence d'un adaptateur de transition (700) à l'intérieur du récepteur de cartouche (400), et une mise en prise d'une pointe capillaire (651) du tube capillaire (650) avec l'adaptateur de transition (700) à l'intérieur du récepteur de cartouche (400) de l'unité de libération d'aérosol (130) lors du déplacement de la partie active de la cartouche (600) d'une extrémité proximale du récepteur de cartouche (400) jusqu'à une extrémité distale du récepteur de cartouche (400).
